# EUROPEAN PATENT APPLICATION

(11) **EP 4 360 645 A1**
(43) Date of publication of application: **01.05.2024**
(21) Application number: 22827705.9
(22) Date of filing: 24.06.2022
(51) Int. Cl.: A61K 38/26, A61K 9/20, A61P 3/10, A61P 3/04

(54) **PHARMACEUTICAL COMPOSITION CONTAINING GLP-1 COMPOUND**

(30) Priority: 25.06.2021 CN 202110709288
(71) Applicant: GAN & LEE PHARMACEUTICALS CO., LTD., Beijing 101109 (CN)
(72) Inventor: ZHANG, Yining, Beijing 101109 (CN); YU, Yong, Beijing 101109 (CN); CHEN, Wei, Beijing 101109 (CN); JI, Fangzhou, Beijing 101109 (CN); ZHANG, Man, Beijing 101109 (CN); WANG, Yongchun, Beijing 101109 (CN); XUE, Fangkai, Beijing 101109 (CN); NIU, Jianghong, Beijing 101109 (CN)
(74) Representative: Kraus & Lederer PartGmbB
(86) International application number: PCT/CN2022/101272
(87) International publication number: WO 2022/268213

(57) **Abstract**

The present invention relates to a pharmaceutical composition of a GLP-1 compound and a preparation method therefor. In particular, the present invention relates to a pharmaceutical composition comprising a GLP-1 compound and salt of N-(8-(2-hydroxybenzoyl)amino)caprylic acid (NAC). The pharmaceutical composition has excellent efficacy, good bioavailability, excellent dissolution, and satisfactory physical stability and chemical stability.

## Description

### Cross-Reference to Related Applications

This application claims priority to Chinese Patent Application No. CN202110709288.X, filed on June 25, 2021, which is incorporated herein by reference in its entirety.

### Technical Field

The present invention discloses a pharmaceutical composition comprising a GLP-1 compound and a salt of N-(8-(2-hydroxybenzoyl)amino)caprylic acid (NAC), as well as methods for preparing the pharmaceutical composition and its use in medicine.

### Background Art

Glucagon-like peptide-1 (GLP-1) and its analogues and derivatives have been found to be highly effective in the treatment of type 1 and type 2 diabetes. However, the high clearance rate limits the effectiveness of these compounds. Various approaches have been used to modify the structure of GLP-1 to provide compounds with longer duration of action in the body. For example, WO99/43708 discloses GLP-1 (7-35) and GLP-1 (7-36) derivatives with lipophilic substitutions attached to C-terminus amino acid residues. WO00/34331 discloses acylated GLP-1 analogues. WO00/69911 discloses activated proinsulin peptides for injection into patients.

Currently marketed GLP-1 drugs include Exenatide administered twice daily, Liraglutide and Lixisenatide administered once daily, and Semaglutide, Abiglutide, Dulaglutide, and Polyethylene glycol loxenatidc administered once weekly. However, these products are predominantly injectable formulations, which can cause discomfort to patients, especially with multiple daily injections, leading to reduced patient compliance. In addition, the use of injectable formulations requires a high level of injection technique proficiency, complex manufacturing processes, and high production costs. Thus, oral administration is the most widely used and patient-friendly route of administration, especially for long-term treatment, and it has lower sterility requirements and lower production costs compared to injectable formulations. However, due to the poor permeability of GLP-1 compounds across the gastrointestinal membrane, oral administration results in very low bioavailability of GLP-1 compounds.

WO2013/139694 discloses a pharmaceutical composition of a salt of GLP-1 peptide and N-(8-(2-hydroxybenzoyl)amino)caprylic acid (NAC), and a method for preparing the pharmaceutical composition, which involves separate granulation of GLP-1 peptide and NAC salt, followed by blending and tablet compression. However, the separate granulation hinders the thorough mixing of GLP-1 peptide and NAC salt, resulting in a pharmaceutical composition with uneven mixing.

Currently, the only oral GLP-1 analogue product on the market is semaglutide tablets (RYBELSUS), which uses SNAC as the salt of N-(8-(2-hydroxybenzoyl)amino)caprylic acid (NAC). It provides some protection for the active substance semaglutide from degradation and promotes its transmembrane absorption. However, the presence of non-proteinogenic amino acids in semaglutide may pose unknown potential risks and the clinical studies have shown its bioavailability to be around 1%, leaving the room for further improvement.

Therefore, there is still a need for an oral GLP-1 formulation with better or equivalent efficacy or therapeutic effect, better or equivalent weight reduction effect, longer or equivalent half-life, faster or equivalent dissolution rate, higher or equivalent bioavailability, and better or equivalent physicochemical stability compared to the oral semaglutide tablets on the market.

### Disclosure of Invention

To overcome or improve at least one disadvantage of the prior art or to provide useful alternatives, the present invention provides a novel pharmaceutical composition of GLP-1. The novel pharmaceutical composition exhibits better or equivalent efficacy or therapeutic effect, better or equivalent weight reduction effect, longer or equivalent half-life, faster or equivalent dissolution rate, higher or equivalent bioavailability, and better or equivalent physicochemical stability compared to the marketed semaglutide tablets.

In the first aspect of the present invention, a pharmaceutical composition is provided comprising a GLP-1 compound and a salt of N-(8-(2-hydroxybenzoyl)amino)caprylic acid (NAC), wherein the GLP-1 compound is a compound of formula (B), or a pharmaceutically acceptable salt, amide, or ester thereof:

[Acy-(L1)ᵣ(L2)_{q}]-G1 (B),

wherein G1 is a GLP-1 analogue having Arg and Ala or Gly respectively at positions corresponding to position 34 and position 8, respectively, of GLP-1 (7-37) (SEQ ID NO: 1), and [Acy-(L1)ᵣ-(L2)_{q}] is a substituent linked to the ε-amino group of the Lys residue at position 26 of the GLP-1 analogue, wherein
r is an integer from 1 to 10, and q is 0 or an integer from 1 to 10;
Acy is a fatty diacid containing 20-24 carbon atoms, wherein formally the hydroxyl group is removed from one of the carboxyl groups of the fatty diacid;
L1 is an acid residue selected from the following group consisting of γGlu, aGlu, βAsp, αAsp, γ-D-Glu, α-D-Glu, β-D-Asp, and α-D-Asp;
L2 is a neutral amino acid residue containing alkylene glycol;
Acy, L1, and L2 are connected by an amide bond; and
the order of L1 and L2 presented in formula (B) can be independently interchanged.

In one example, G1 is [Gly8, Arg34]GLP-1-(7-37) peptide (SEQ ID NO: 2) or [Arg34]GLP-1-(7-37) peptide (SEQ ID NO: 3), preferably [Gly8, Arg34]GLP-1-(7-37) peptide.

In another example, r is an integer selected from 1, 2, 3, 4, 5, or 6, preferably 1, 2, 3, or 4, preferably 1 or 2, preferably 1.

In another example, q is 0, 1, 2, 3, 4, 5, 6, 7, or 8, preferably 0, 1, 2, 3, or 4, preferably 0, 1, or 2.

In another example, Acy is a fatty diacid containing 20-23 carbon atoms, preferably Acy is a fatty diacid containing 20, 21, or 22 carbon atoms, wherein a hydroxyl group is removed from one of the carboxyl groups of fatty diacid.

In one example, L2 is: -HN-(CH₂)₂-O-(CH₂)₂-O-CH₂-CO-, -HN-(CH₂)₂-O-(CH₂)₂-O-(CH₂)₂-O-(CH₂)₂-O-(CH₂)₂-CO- , -HN-(CH₂)₂-O-(CH₂)₂-O-(CH₂)₂-O-(CH₂)₂-O-(CH₂)₂-O-(CH₂)₂-O-(CH₂)₂-CO- , -HN-(CH₂)₂-O-(CH₂)₂-O-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₃-O-(CH₂)₄-O-(CH_{z})₃-NH-CO-, -HN-(CH₂)₃-O-(CH₂)₄-O-(CH₂)₃-NH-CO-CH₂-O-CH₂-CO-, -HN-(CH₂)₃-O-(CH₂)₄-O-(CH₂)₃-NH-CO-(CH₂)₂-CO-, -HN-(CH₂)₂-O-(CH₂)₂-O-CH₂-CO-CH₂-O-CH₂-CO-, -HN-(CH₂)₃-O-(CH₂)₂-O-(CH₂)₂-O-(CH₂)₃-NH-CO-(CH₂)₂-CO- , -HN-(CH₂)₃-O-(CH₂)₂-O-(CH₂)₂-O-(CH₂)₃-NH-CO-CH₂-O-CH₂-CO-, -HN-(CH₂)₂-O-(CH₂)₂-O-(CH₂)₂-NH-CO-(CH₂)₂-CO-, -HN-(CH2)₂-O-(CH₂)₂-O-(CH₂)₂-NH-CO-CH₂-O-CH₂-CO-, -HN-(CH₂)₃-O-(CH₂)₂-O-(CH₂)₂-O-(CH₂)₃-NH-CO-CH₂-O-CH₂-CO-, -HN-(CH₂)₃-O-(CH₂)₃-O-CH₂-CO-, or -HN-(CH₂)₄-O-(CH₂)₄-O-CH₂-CO-; preferably L2 is -HN-(CH₂)₂-O-(CH₂)₂-O-CH₂-CO-.

In another example, L1 is selected from γGlu or βAsp, preferably γGlu.

In another example, Acy is HOOC-(CH₂)ₗₛ-CO-, HOOC-(CH₂)₁₉-CO-, HOOC-(CH₂)₂₀-CO-, HOOC-(CH₂)₂₁-CO- or HOOC-(CH₂)₂₂-CO-, preferably, Acy is HOOC-(CH₂)₁₈-CO-, HOOC-(CH₂)₂₀-CO- or HOOC-(CH₂)₂₂-CO-.

In one example, Acy, L1, and L2 in formula (B) are sequentially connected by amide bonds, and the C-terminus of L2 is connected to the ε-amino group of the Lys residue at position 26 of the GLP-1 analogue.

In one example, the GLP-1 compound in the pharmaceutical composition of the first aspect of the present invention is selected from the following compounds:
*N*-ε²⁶-[2-(2-[2-(2-[2-(2-[4-(19-carboxynonadecanoylamino)-4(*S*)-carboxybutanoylarnino]ethoxy)ethoxy]acetylamino)ethoxy]ethoxy)acetyl][Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly⁸, Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl] [Gly8, Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Gly8, Arg34]GLP-1-(7-37) peptide,
*N*-ε²⁶-(23-carboxytricosanoylamino)-4(*S*)-carboxybutanoyl-[Gly8, Arg34]GLP-1-(7-37) peptide,
N-ε²⁶-(19-carboxynonadecanoylamino)-4(*S*)-carboxybutanoyl-[Gly8, Arg34]GLP-1-(7-37) peptide,
*N*-ε²⁶-(21-carboxyheneicosanoylamino)-4(S)-carboxybutanoyl-[Gly8, Arg34]GLP-1-(7-37) peptide,
*N*-e²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Arg34]GLP-1-(7-37) peptide,
*N-*ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl] [Arg34]GLP-1 -(7-37) peptide,
*N*-ε²⁶-[2-(2-[2-(2-[2-(2-[4-(23-carboxytricosanoylainino)-4(*S*)-carboxybutanoylamino]ethoxy)ethoxy]acetylamino)ethoxy]ethoxy)acetyl] [Arg34]GLP-1 -(7-37) peptide,
*N*-ε²⁶-[2-(2-[2-(4-[23-carboxytricosanoylamino]-4(S)-carboxybutanoylamino)ethoxy]ethoxy)acetyl][Arg34]GLP-1-(7-37) peptide,
*N*-e²⁶-(23-carboxytricosanoylamino)-4(*S*)-carboxybutanoyl-[Arg34]GLP-1-(7-37) peptide,
*N-*ε²⁶-(19-carboxynonadecanoylamino)-4(*S*)-carboxybiitanoyl-[Arg34]GLP-1-(7-37) peptide,
*N*-ε²⁶-(21-carboxyheneicosanoylamino)-4(*S*)-carboxybutanoyl-[Arg34]GLP-1-(7-37) peptide,
*N-*ε²⁶-[2-(2-[2-(2-[2-(2-[4-(20-carboxyeicosanoylamino)-4(*S*)-carboxybutanoylamino]ethoxy)ethoxyJacetylamino)ethoxy]ethoxy)acetyl] [Gly8, Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Gly8, Arg34]GLP-1-(7-37) peptide,
*N*-ε²⁶-(20-carboxyeicosanoylamino)-4(*S*)-carboxybutanoyl-[Gly8, Arg34]GLP-1-(7-37) peptide,
*N*-ε²⁶-(22-carboxydocosanoylamino)-4(*S*)-carboxybutanoyl-[Gly8, Arg34]GLP-1-(7-37) peptide,
*N*-ε²⁶-[2-(2-[2-(2-[2-(2-[4-(20-carboxyeicosanoylammo)-4(*S*)-carboxybutanoylamino]ethoxy)ethoxy]acetylamino)ethoxy]ethoxy)acetyl][Arg34]GLP-1-(7-37) peptide,
*N-*ε²⁶-[2-(2-[2-(4-[20-carboxyeicosanoylamino]-4(*S*)-carboxybutanoylamino)ethoxy]ethoxy)acetyl][Arg34]GLP-1-(7-37) peptide,
*N*-s²⁶-[2-(2-[2-(2-[2-(2-[4-(22-carboxydocosanoylainino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Arg34]GLP-1-(7-37) peptide,
*N*-ε²⁶-(20-carboxyeicosanoylamino)-4(*S*)-carboxybutanoyl-[Arg34]GLP-1-(7-37) peptide, and
   *N*-ε²⁶-(22-carboxydocosanoylamino)-4(*S*)-carboxybutanoyl-[Arg34]GLP-1-(7-37) peptide.

In one example, the GLP-1 compound in the pharmaceutical composition of the first aspect of the present invention is selected from the following compounds:
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl] [Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
*N-*ε²⁶-(19-carboxynonadecanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl-[Arg34]GLP-1-(7-37) peptide, *N*-ε²⁶-[2-(2-[2-(2-[2-(2-[4-(21-carboxyheneicosanoylamino)-4(*S*)-carboxybutanoylamino]ethoxy)cthoxy]acetylamino)ethoxy]ethoxy)acetyl[Gly8, Arg34]GLP-1-(7-37) peptide, and
*N*-ε²⁶-(2-[2-(4-[21-carboxyheneicosanoylamino]-4(*S*)-carboxybutanoylamino)ethoxy]ethoxy)acetyl][Gly8, Arg34]GLP-1-(7-37) peptide.

The inventors unexpectedly discovered that the GLP-1 pharmaceutical composition of the present invention, when the GLP-1 compound of formula (B) described above is used as an active ingredient, exhibits better or comparable therapeutic or pharmacological effects, better or comparable weight-reducing effects, longer or comparable half-life, faster or comparable dissolution rate, higher or comparable bioavailability, and better or comparable physicochemical stability compared to the marketed semaglutide tablets.

In one example, the salt of N-(8-(2-hydroxybenzoyl)amino)caprylic acid (NAC) is the sodium salt (SNAC) of N-(8-(2-hydroxybenzoyl)amino)caprylic acid.

In one example, the composition comprises one or more pharmaceutically acceptable excipients.

In one example, the pharmaceutically acceptable excipients are selected from one or more from the group consisting of glidants, binders, fillers, disintegrants, or lubricants.

In one example, the glidants are selected from talc and colloidal silica, preferably colloidal silica.

In another example, the binders are selected from polyvinylpyrrolidone, copovidone, and hydroxypropyl cellulose, preferably polyvinylpyrrolidone, and more preferably polyvinylpyrrolidone K90.

In another example, the fillers are selected from the one or more from the group consisting of microcrystalline cellulose, cellulose powder, dicalcium phosphate, corn starch, pregelatinized starch, anhydrous lactose, mannitol, erythritol, sucrose, sorbitol, calcium phosphate and dextrin, preferably microcrystalline cellulose or anhydrous lactose, and more preferably microcrystalline cellulose.

In another example, the lubricants are selected from one or more from the group consisting of magnesium stearate, magnesium lauryl sulfate, stearic acid, sodium octadecyl fumarate, or glyceryl tribehenate, preferably magnesium stearate.

In an example, the formulation comprises at least about 50% (w/w), preferably at least about 55% (w/w), preferably at least about 60% (w/w), preferably about 50%-90% (w/w), preferably about 55%-85% (w/w), preferably about 60%-80% (w/w), preferably about 61% (w/w), about 62% (w/w), about 63% (w/w), about 64% (w/w), about 65% (w/w), about 66% (w/w), about 67% (w/w), about 68% (w/w), about 69% (w/w), about 70% (w/w), about 71% (w/w), about 72% (w/w), about 73% (w/w), about 74% (w/w), about 75% (w/w), about 76% (w/w), about 77% (w/w), about 78% (w/w), or about 79% (w/w) of NAC salt, preferably, wherein said NAC salt is SNAC.

In an example, the formulation comprises no more than 25%, preferably no more than 20% (w/w), preferably no more than 17% (w/w), preferably no more than 15% (w/w), preferably about 0.5%-20% (w/w), preferably about 0.5%-18.5% (w/w), preferably about 0.5%-16.5% (w/w), preferably about 0.5%-15% (w/w), preferably about 0.5%-13% (w/w), preferably about 0.5%-12% (w/w), preferably about 0.5%-11% (w/w), preferably about 0.6%-11% (w/w), preferably about 0.6%-10% (w/w), preferably about 0.6%-9% (w/w), preferably about 0.6%-8% (w/w), preferably about 0.6%-7% (w/w), preferably about 0.6%-6% (w/w), preferably about 0.7%-5% (w/w), preferably about 1.20/0-11% (w/w), preferably about 1.2%-10% (w/w), preferably about 1.2%-9% (w/w), preferably about 1.2%-8% (w/w), preferably about 1.2%-7% (w/w), preferably about 1.2%-6% (w/w), preferably about 1.20/0-5% (w/w), preferably about 1.2%-4.5% (w/w), preferably about 1.4%-4% (w/w), preferably about 1.4%-3.5% (w/w), preferably about 1.40/0-3% (w/w), preferably about 1.5% (w/w), about 1.7% (w/w), about 2% (w/w), about 2.5% (w/w), or about 2.8% (w/w) of said GLP-1 compound, preferably wherein the GLP-1 compound is selected from *N-*ε²⁶-[2-(2-[2-(2-[2-(2-[4-(19-carboxynonadecanoylamino)-4(S)-carboxybutanoylamino]ethoxy)ethoxy]acetylamino)ethoxy]ethoxy)acetyl] [Gly8, Arg34]GLP-1-(7-37) peptide,
*N*-ε²⁶-[2-(2-[2-{4-[19-carboxynonadecanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(21-carboxyheneicosanoylamino)-4(8)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl] [Gly8, Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Gly8, Arg34]GLP-1-(7-37) peptide,
*N*-ε²⁶-(23-carboxytricosanoylamino)-4(*S*)-carboxybutanoyl-[Gly8, Arg34]GLP-1-(7-37) peptide,
*N*-ε²⁶-(1 9-carboxynonadecanoylmnino)-4(*S*)-carboxybutanoyl-[Gly8, Arg34]GLP-1-(7-37) peptide,
*N*-ε²⁶-(21-carboxyheneicosanoylamino)-4(*S*)-carboxybutanoyl-[Gly8, Arg34]GLP-1 -(7-37) peptide,
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl] [Arg34]GLP-1 -(7-37) peptide,
*N*-ε²⁶-[2-(2-[2-(4-[19-carboxynonadecanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Arg34]GLP-1 -(7-37) peptide,
*N*-ε²⁶-(23-carboxytricosanoylamino)-4(*S*)-carboxybutanoy1-[Arg34]GLP-1-(7-37) peptide,
*N*-ε²⁶-(19-carboxynonadecanoylamino)-4(*S*)-carboxybiitanoyl-[Arg34]GLP-1-(7-37) peptide,
*N*-ε²⁶-(21-carboxyheneicosanoylamino)-4(*S*)-carboxybutanoyl-[Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl] [Gly8, Arg34]GLP-1 - (7-37) peptide,
*N*-ε²⁶-[2-(2-[2-(4-[20-carboxyeicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Gly8, Arg34]GLP-1-(7-37) peptide,
*N*-ε²⁶-(20-carboxyeicosanoylamino)-4(*S*)-carboxybutanoyl-[Gly8, Arg34]GLP-1-(7-37) peptide,
*N*-ε²⁶-(22-carboxydocosanoylamino)-4(*S*)-carboxybutanoyl-[Gly8, Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Arg34]GLP-1-(7-37) peptide,
*N*-ε²⁶-(20-carboxyeicosanoylamino)-4(*S*)-carboxybutanoyl-[Arg34]GLP-1-(7-37) peptide, and
   *N*-ε²⁶-(22-carboxydocosanoylamino)-4(*S*)-carboxybutanoyl-[Arg34]GLP-1-(7-37) peptide.

In one example, the composition comprises about 0.1%-20% (w/w), preferably about 0.2%-10% (w/w), more preferably about 0.3%-5% (w/w), about 0.5%-3% (w/w), about 0.7%-2% (w/w), about 1%-2% (w/w), about 1.6% (w/w)-about 2.0% (w/w), more preferably about 1.6% (w/w), about 1.7% (w/w), about 1.8% (w/w), about 1.9% (w/w), or about 2.0% (w/w) of a binder. The preferred binder is selected from polyvinylpyrrolidone, copovidone, and hydroxypropyl cellulose, with polyvinylpyrrolidone being the preferred binder, and polyvinylpyrrolidone K90 being more preferred.

In another example, the composition comprises about 5%-40% (w/w), preferably about 10%-35% (w/w), more preferably about 10%-30% (w/w), about 10%-25% (w/w), about 130/0-17% (w/w), about 13% (w/w), about 14% (w/w), about 15% (w/w), about 16% (w/w), about 17% (w/w), or about 20% (w/w) of a filler. The filler is selected from one or more of microcrystalline cellulose, cellulose powder, dicalcium phosphate, corn starch, pregelatinized starch, anhydrous lactose, mannitol, erythritol, sucrose, sorbitol, calcium phosphate, and dextrin, preferably microcrystalline cellulose or anhydrous lactose, and more preferably microcrystalline cellulose.

In another example, the composition comprises about 0.1%-10% (w/w) preferably about 0.5%-10% (w/w), more preferably about 0.5%-5% (w/w), about 0.5%-3.5% (w/w), about 2.8% (w/w), about 2.9% (w/w), about 3% (w/w), about 3.1% (w/w), about 3.2% (w/w), about 3.3% (w/w), about 3.4% (w/w), or about 3.5% (w/w) of a lubricant. The lubricant is selected from one or more of magnesium stearate, magnesium lauryl sulfate, stearic acid, sodium stearyl fumarate, and glyceryl tribehenate, preferably magnesium stearate.

In another example, the composition comprises about 0.1%-20% (w/w), preferably about 0.2%-10% (w/w), more preferably about 0.3%-5% (w/w), about 0.5%-3% (w/w), about 0.7%-2% (w/w), about 1%-2% (w/w), about 1.6%-2% (w/w), about 1.6% (w/w), about 1.7% (w/w), about 1.8% (w/w), about 1.9% (w/w), or about 2% (w/w) of the glidant. The glidants are selected from talc and colloidal silica, preferably colloidal silica.

In one example, the composition comprises about 1-100mg, preferably about 2-90mg, preferably about 3-80mg, about 4-60mg, about 5-55mg, about 5-50mg, about 5-45mg, about 5-40mg, about 5-35mg, about 5-30mg, about 5-25mg, about 5-20mg, about 5-15mg, about 5-10mg, about 7-50mg, about 7-30mg, about 7-25mg, about 7-20mg, about 2mg, about 3mg, about 4mg, about 5mg, about 6mg, about 7mg, about 8mg, about 9mg, about 10mg, about 14mg, about 25mg, or about 50mg of the GLP-1 compound. Preperably, the GLP-1 compound is selected from
*N*-ε²⁶-[2-(2-[2-(2-[2-(2-[4-(19-carboxynonadecanoylamino)-4(*S*)-carboxybutanoylamino]ethoxy)cthoxy]acetylamino)ethoxy]cthoxy)acetyl][Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]cthoxy)acetyl] [Gly 8, Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Gly8, Arg34]GLP-1-(7-37) peptide,
*N*-ε²⁶-(23-carboxytricosanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(21-carboxyheneicosanoylamino-4(*S*)carboxybutanoyl-[Gly8, Arg34]GLP-1-(7-37) peptide,
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Arg34]GLP-1-(7-37) peptide, *N*-ε²⁶-[2-(2-[2-(2-[242-[4-(21-carboxyheneicosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Arg34]GLP-I-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl]Arg34]GLP-1-(7-37) peptide,
-*N*-ε²⁶[2-(2-[2-(4-[23-carboxytricosanoylamino]-4(*S*)-carboxybutanoylamino)ethoxy]ethoxy)acetyl][Arg34]GLP-1-(7-37) peptide,
*N*-ε²⁶-(23-carboxytricosanoylamino)-4(*S*)-carboxybutanoyl-[Arg34]GLP-1-(7-37) peptide,
*N*-ε²⁶-(19-carboxynonadecanoylamino)-4(*S*)-carboxybutanoyl-[Arg34]GLP-1-(7-37) peptide,
*N*-ε²⁶-(21-carboxyheneicosanoylamino)-4(*S*)-carboxybutanoyl-[Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Gly8, Arg34]GLP-1-(7-37) peptide,
*N*-ε²⁶-(20-carboxyeicosanoylamino)-4(*S*)-carboxybutanoyl-[Gly8, Arg34]GLP-1-(7-37) peptide,
*N*-ε²⁶-(22-carboxydocosanoylamino)-4(*S*)-carboxybutanoyl-[Gly8, Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Arg34]GLP-1-(7-37) peptide, *N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Arg34]GLP-1-(7-37) peptide,
*N*-ε²⁶-(20-carboxyeicosanoylamino)4(*S*)-carboxybutanoyl-[Arg34]GLP-1-(7-37) peptide, and
   *N*-ε²⁶-(22-carboxydocosanoylamino)-4(*S*)-carboxybutanoyl-[Arg34]GLP-1-(7-37) peptide.

In another example, the composition comprises at least about 50mg, preferably at least about 100mg, more preferably at least about 150mg, about 50-600mg, about 100-500mg, about 150-450mg, about 175-425mg, about 200-400mg, about 250-400mg, more preferably about 300mg, about 350mg, about 400mg of the salt of NAC, wherein the NAC salt is SNAC.

In another example, the composition comprises about 0.5-50mg, preferably about 1-40mg, preferably about 1-30ing, about 1-25mg, about 1-20mg, about 1-15mg, about 2-14mg, about 3-13mg, about 4-12mg, about 5-11mg, about 6-10mg, about 7-9mg, about 8mg of a binder. The binders are selected from polyvinylpyrrolidone, copovidone, and hydroxypropyl cellulose, preferably polyvinylpyrrolidone, and more preferably polyvinylpyrrolidone K90.

In another example, the composition comprises about 10-150mg, preferably about 20-140mg, more preferably about 30-130mg, about 40-120ing, about 50-110mg, about 50-100mg, about 60-90mg, about 60-80mg, about 60-70mg, about 68mg of a filler. The fillers are selected from microcrystalline cellulose, cellulose powder, calcium hydrogen phosphate, corn starch, pregelatinized starch, anhydrous lactose, mannitol, erythritol, sucrose, sorbitol, calcium phosphate, and dextrin, preferably microcrystalline cellulose or anhydrous lactose, and more preferably microcrystalline cellulose.

In another example, the composition comprises about 1-50mg, preferably about 1-40mg, more preferably about 1-30mg, about 2-20mg, about 3-20mg, about 5-20mg, about 5-15mg, about 10-15mg, about 14mg of a lubricant. The lubricants are selected from magnesium stearate, magnesium lauryl sulfate, stearic acid, sodium stearyl fumarate, and glyceryl tribehenate, preferably magnesium stearate.

In another example, the composition comprises about 1-50mg, preferably about 140mg, more preferably about 1-30mg, about 2-20mg, about 3-20mg, about 5-15mg, about 5-10mg, about 8mg of a glidant. The glidants are selected from talc and colloidal silica, preferably colloidal silica.

In one example, the pharmaceutical composition is in the form of a tablet, granule, or capsule formulation, preferably a tablet formulation.

In one example, the hardness of the composition is less than about 130N, preferably about 80-130N, preferably about 90-130N, preferably about 100-125N, and more preferably about 110-120N.

In one example, the particle size D90 of the composition is preferably not exceeding about 200µm, more preferably not exceeding about 130µm, preferably being about 15-130µm, and more preferably being about 15-50pm.

In one example, the pharmaceutical composition protected by the first aspect of the present invention comprises the following components:
About 5, 10, 15, 20, 25, 30, 35, 40, 45, 50, 55, 60, 70, 80, 90, or 100mg, preferably about 7-10mg of *N*-ε²⁶-[2-(2-[2-(2-[2-(2-[4-(21-carboxyl dococanoyl amino)-4(*S*)-carboxybutyryl amino]ethoxy)ethoxy]acetyl amino)ethoxy]ethoxy) acetyl] [Gly8, Arg34]GLP-1-(7-37) peptide; about 300, 350, or 400mg of SNAC; about 8mg of polyvinylpyrrolidone K90; about 68mg of microcrystalline cellulose; about 14mg of magnesium stearate; and about 8mg of colloidal silica.

In one example, the total weight of the pharmaceutical composition is about 150-1000mg, preferably about 175-1000mg, more preferably about 200-800mg, about 400mg-500mg, preferably about 400mg, about 403mg, about 405mg, about 408mg, about 413mg, about 418mg, about 423mg, about 428mg, about 433mg, about 438mg, about 443mg, about 448mg, about 455mg, about 500mg, about 600mg, or about 800mg.

The second aspect of the present invention provides a preparation method for preparing the pharmaceutical composition according to the first aspect of the present invention.

In one example, the preparation method includes the step of mixing the GLP-1 compound with the salt of NAC for one-time granulation.

In one example, the preparation method includes mixing the salt of NAC with a lubricant and/or a glidant before mixing it with the GLP-1 compound.

In one example, the preparation method includes the following steps:
(1) Mixing the salt of NAC, the glidant, and a portion of the lubricant to obtain a premix powder A;
(2) Mixing the GLP-1 compound, the binder, and the filler with the premix powder A to obtain a premix powder B;
(3) Granulating the premix powder B, preferably by dry granulation;
(4) Mixing the obtained granules with the remaining lubricant to obtain a blend of granules;
(5) Compression the blend of granules into tablets.

In one example, the GLP-1 compound is selected from
*N*-ε²⁶-[2-(2-[2-(2-[2-(2-[4-(19-carboxynonadecanoylamino)-4(*S*)-carboxybutanoy]amino]ethoxy)ethoxy]acetylamino)ethoxy]ethoxy)acetyl][Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)elhoxy]acetylamino)ethoxy]ethoxy)acetyl][Gly8, Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Gly8, Arg34]GLP-1-(7-37) peptide,
*N*-ε²⁶-(23-carboxytricosanoylamino)-4(*S*)-carboxybutanoyl-[Gly8, Arg34]GLP-1-(7-37) peptide,
*N-*ε²⁶-(19-carboxynonadecanoylamino)-4(*S*)-carboxybutanoyl-[Gly8, Arg34]GLP-1-(7-37) peptide,
*N*-ε²⁶-(21-carboxyheneicosanoylamino)-4(*S*)-carboxybutanoyl-[Gly8, Arg34]GLP-1-(7-37) peptide,
*N*-ε²⁶-[2-(2-[2-(2-[2-(2-[2-(19-carboxynonadecanoylamino)-4(8)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)cthoxy]acetytamino)ethoxy]ethoxy)acetyt][Arg34]GLP-1-(7-37) peptide,
*N*-ε²⁶-[2-(2-[2-(4-[21-carboxyheneicosanoylamino]-4(5)-carboxybutanoylamino)ethoxy]ethoxy)acetyl][Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Arg34]GLP-1-(7-37) peptide, *N*-ε²⁶-(23-carboxytricosanoylamino)-4(*S*)-carboxybutanoyl-[Arg34]GLP-1-(7-37) peptide,
*N*-ε²⁶-(19-carboxynonadecanoylamino)-4(*S*)-carboxybutanoyl-[Arg34]GLP-1-(7-37) peptide,
*N*-ε²⁶-(21-carboxyheneicosanoylamino)-4(*S*)-carboxybutanoyl-[Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Gly8, Arg34]GLP-1-(7-37) peptide,
*N*-ε²⁶-(20-carboxyeicosanoylamino)-4(*S*)-carboxybutanoyl-[Gly8, Arg34]GLP-1-(7-37) peptide,
*N*-ε²⁶-(22-carboxydocosanoylamino)-4(*S*)-carboxybutanoyl-[Gly8, Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Arg34]GLP-1-(7-37) peptide,
*N*-ε²⁶-(20-carboxyeicosanoylamino)-4(*S*)-carboxybutanoyl-[Arg34]GLP-1-(7-37) peptide, and
   *N*-ε²⁶-(22-carboxydocosanoylamino)-4(*S*)-carboxybutanoyl-[Arg34]GLP-1-(7-37) peptide.

In another example, the salt of NAC is SNAC; and/or
The binder is selected from polyvinylpyrrolidone, copovidone, and hydroxypropyl cellulose, preferably polyvinylpyrrolidone, and more preferably polyvinylpyrrolidone K90; and/or

The filler is selected from one of more of microcrystalline cellulose, cellulose powder, calcium hydrogen phosphate, corn starch, pregelatinized starch, anhydrous lactose, mannitol, erythritol, sucrose, sorbitol, calcium phosphate, and dextrin, preferably microcrystalline cellulose or anhydrous lactose, and more preferably microcrystalline cellulose.

In one example, the lubricant is selected from one of more of magnesium stearate, magnesium lauryl sulfate, stearic acid, sodium stearyl fumarate, and glyceryl tristearate, preferably magnesium stearate; and/or

The glidant is selected from talc and colloidal silica, preferably colloidal silica.

Another aspect of the present invention relates to the aforementioned pharmaceutical composition, which is used as a medicament.

Another aspect of the present invention relates to the aforementioned pharmaceutical composition for use in the treatment or prevention of hyperglycemia, diabetes, and/or obesity.

Another aspect of the present invention relates to the use of the aforementioned pharmaceutical composition in the preparation of a medicament for the treatment or prevention of hyperglycemia, diabetes, and/or obesity.

Another aspect of the present invention provides a method for the treatment or prevention of hyperglycemia, diabetes, and/or obesity, comprising administering an effective amount of the aforementioned pharmaceutical composition.

### Description of the Figures

Figure 1a shows the hypoglycemic effects of the pharmaceutical compositions containing Compound 5 and SNAC, the pharmaceutical compositions containing semaglutide and SNAC, and the solvent on KM mice.
Figure 1b, in correspondence with the Figure 1a, shows the AUC (area under the curve) of the hypoglycemic effects of the pharmaceutical compositions containing Compound 5 and SNAC, the pharmaceutical compositions containing semaglutide and SNAC, and the solvent on KM mice.
Figure 1c shows the control effect of the food intake of KM mice by the pharmaceutical compositions containing Compound 5 and SNAC, the pharmaceutical compositions containing semaglutide and SNAC, and the solvent.
Figure 2a shows the hypoglycemic effects of the pharmaceutical compositions containing Compound 5 and SNAC, the pharmaceutical compositions containing semaglutide and SNAC, and the solvent on db/db mice.
Figure 2b, in correspondence with the Figure 2a, shows the AUC of the hypoglycemic effects of the pharmaceutical compositions containing Compound 5 and SNAC, the pharmaceutical compositions containing semaglutide and SNAC, and the solvent on db/db mice.
Figure 3a shows the hypoglycemic effects of the pharmaceutical compositions containing Compound 5 and SNAC, the pharmaceutical compositions containing semaglutide and SNAC, and the solvent on SD rats.
Figure 3b, in correspondence with the Figure 3a, shows the AUC of the hypoglycemic effects of the pharmaceutical compositions containing Compound 5 and SNAC, the pharmaceutical compositions containing semaglutide and SNAC, and the solvent on SD rats.
Figure 4a shows the hypoglycemic effects of the pharmaceutical compositions containing Compound 5 and SNAC, the pharmaceutical compositions containing semaglutide and SNAC, and the solvent on ICR mice.
Figure 4b, in correspondence with the Figure 4a, shows the AUC of the hypoglycemic effects of the pharmaceutical compositions containing Compound 5 and SNAC, the pharmaceutical compositions containing semaglutide and SNAC, and the solvent on ICR mice.
Figure 5a shows the hypoglycemic effects of the title compounds of Example 5 of the present invention, the title compounds of Example 2, and the solvent on Kkay mice.
Figure 5b, in correspondence with the Figure 5a, shows the AUC of the hypoglycemic effects of the title compounds of Example 5 of the present invention, the title compounds of Example 2, and the solvent on Kkay mice.
Figure 5c shows the effect of reducing HbAlc levels in Kkay mice by the title compounds of Example 5 of the present invention, the title compounds of Example 2, and the solvent.
Figure 6a shows the hypoglycemic effects of the pharmaceutical compositions containing Compound 5 and SNAC, and the solvent on db/db mice or normal mice.
Figure 6b, in correspondence with the Figure 6a, shows the AUC of the hypoglycemic effects of the pharmaceutical compositions containing Compound 5 and SNAC, and the solvent on db/db mice or normal mice.
Figure 7a shows the hypoglycemic effects of the pharmaceutical compositions containing Compound 5 and SNAC, and the solvent on db/db mice.
Figure 7b, in correspondence with the Figure 7a, shows the AUC of the hypoglycemic effects of the pharmaceutical compositions containing Compound 5 and SNAC, and the solvent on db/db mice.
Figure 7c shows the weight reduction effect in db/db mice by the pharmaceutical compositions containing Compound 5 and SNAC, and the solvent
Figure 7d shows the food intake inhibition effect in db/db mice by the pharmaceutical compositions containing Compound 5 and SNAC, and the solvent.
Figure 8 shows the weight control effect in SD rats by the pharmaceutical compositions containing Compound 5 and SNAC, and the solvent.

### Detailed Description

### Definition

### GLP-1 analogues and GLP-1 Derivatives

The terms "GLP-1 analogue" or "analog of GLP-1" as used herein refer to peptides or compounds that are variants of the human glucagon-like peptide-1 (GLP-1(7-37)), in which one or more amino acid residues of GLP-1 (7-37) are substituted and/or one or more amino acid residues are deleted, and/or one or more amino acid residues are added. Specifically, the sequence of GLP-1 (7-37) is shown in SEQ ID NO:1 in the sequence listing. Peptides having the sequence of SEQ ID NO:1 can also be referred to as "natural" GLP-1 or "natural" GLP-1(7-37).

In the sequence listing, the first amino acid residue (histidine) of SEQ ID NO:1 is numbered as 1. However, in the following text, according to the established practice in the art, this histidine residue is numbered as 7, and the subsequent amino acid residues are numbered accordingly, ending with glycine at position 37. Therefore, typically, the amino acid residue number or position number of the GLP-1(7-37) sequence referred to in this specification are those starting at position 7 with histidine and ending at position 37 with glycine.

[Gly8, Arg34]GLP-1-(7-37) peptide refers to a GLP-1 analogue in which glycine and arginine are presented at positions 8 and 34, respectively, corresponding to GLP-1(7-37) (SEQ ID NO:1). [Arg34]GLP-1-(7-37) peptide refers to a GLP-1 analogue in which arginine is presented at position 34, corresponding to GLP-1 (7-37) (SEQ ID NO:1). Specifically, the amino acid sequences of [Gly8,Arg34]GLP-1-(7-37) peptide and [Arg34]GLP-1-(7-37) peptide are shown as SEQ ID NO:2 and SEQ ID NO:3, respectively, in the sequence listing.

GLP-1 Peptides or their analogs, as used herein, the term "derivatives" refers to chemically modified GLP-1 peptides or analogs in which one or more substituents are covalently attached to the peptide. Substituents can also be referred to as side chains.

Unless otherwise indicated, when referring to acylation of a lysine residue, it is understood to be carried out with the ε-amino group.

GLP-1 Derivatives of formula (B) of the present invention may exist in different stereoisomeric forms, which have the same molecular formula and the connected atomic sequence but differ only in their three-dimensional orientation in atomic space. Unless otherwise indicated, the present invention encompasses all stereoisomeric forms of the derivatives being protected.

The term "peptide" when used, for example, for GLP-1 analogues of the present invention, refers to a compound that includes a series of amino acids linked together by amide (or peptide) bonds.

In a specific example, the peptide is largely or primarily composed of amino acids connected to each other by amide bonds, for example, at least 50%, 60%, 70%, 80%, or at least 90% by molar mass. In another specific example, the peptide is composed of amino acids connected by peptide bonds.

Amino acids are molecules containing an amino group and a carboxylic acid group, optionally with one or more additional groups, commonly referred to as side chains.

The term "amino acid" includes proteinaceous amino acids (encoded by the genetic code, including natural and standard amino acids), as well as non-proteinaceous (not found in proteins and/or not encoded in the standard genetic code) and synthetic amino acids. Non-proteinaceous amino acids are those that can be incorporated into a peptide by peptide bonds but are not proteinaceous amino acids. Examples of synthetic non-proteinaceous amino acids include amino acids produced by chemical synthesis, i.e., D-isomers of amino acids encoded by the genetic code, such as D-alanine and D-leucine, Aib (a-amino isobutyric acid), Abu (a-amino butyric acid), 3-aminomethylbenzoic acid, ortho-amino benzoic acid, deaminohistidine, β-amino acid analogs of amino acids such as β-alanine, D-histidine, deaminohistidine, 2-aminohistidine, β-hydroxyhistidine, and homohistidine.

Non-limiting examples of non-genetically encoded amino acids include γ-carboxyglutamate, ornithine, D-alanine, D-glutamine, and phosphoserine. Non-limiting examples of non-proteinaceous synthetic amino acids include D-isomers of amino acids, such as D-alanine and D-leucine, Aib (a-amino isobutyric acid), β-alanine, and des-aminohistidine(desH, also known as imidazole propionic acid, abbreviated as Imp).

In the following, all amino acids not identified as optically active isomers are understood to refer to L-isomers (unless otherwise indicated).

### Pharmaceutically acceptable salts, amides, or esters

The GLP-1 derivatives, analogs, and intermediates of the present invention can be in the form of pharmaceutically acceptable salts, amides, or esters. Salts can be basic salts, acid salts, or neutral salts. Basic salts produce hydroxide ions in water, while acid salts produce hydrated hydrogen ions. The salts of the present invention can be formed by reacting with added cations or anions that react with anionic or cationic groups respectively. These groups may be located within the peptide portion and/or in the side chains of the present invention derivatives.

Non-limiting examples of anionic groups for the present invention derivatives include side chains (if present) and free carboxyl groups in the peptide portion. The peptide portion typically includes a free carboxylic acid at the C-terminus, and it may also include free carboxyl groups on internal acidic amino acid residues such as Asp and Glu.

Non-limiting examples of cationic groups in the pepetide portion include free amino groups at the N-terminus (if present) and any free amino groups on internal basic amino acid residues such as His, Arg, and Lys.

Esters of the present invention can be formed, for example, by reacting free carboxyl groups with alcohols or phenols, resulting in the substitution of at least one hydroxyl group by an alkoxy or aryloxy group. The formation of esters can involve free carboxyl at the C-terminus of the peptide and/or any free carboxyl groups on the side chains.

Amides of the present invention can be formed, for example, by reacting free carboxyl groups with amines or substituted amines, or by reacting free or substituted amino groups with carboxylic acids. The formation of amides can involve the free carboxyl at the C-terminus of the peptide, any free carboxyl groups on the side chains, the free amino at the N-tcrminus of the peptide, and/or any free or substituted peptide amino groups in the peptide and/or side chain.

In a specific example, the GLP-1 compound or GLP-1 derivative of the present invention is in the form of a pharmaceutically acceptable salt. In another specific example, it is in the form of a pharmaceutically acceptable amide, preferably having an amide group at the C-terminus of the peptide. In another further specific example, the peptide or derivative is in the form of a pharmaceutically acceptable ester.

The term "amino acid residue" includes amino acids from which hydrogen atoms have been removed from the amino group and/or hydroxyl group has been removed from the carboxyl group and/or hydrogen atoms have been removed from thiol group. Inexact terminology, amino acid residues can be referred to as amino acids.

Unless otherwise indicated, the amino acids mentioned herein are L-amino acids.

Here, the term "alkylene glycol" includes oligo- and polyalkylene glycol portions, as well as monomeric alkylene glycol portions. Monomeric alkylene glycol and polyalkylene glycol include chains based on mono- and polyethylene glycol, mono- and polypropylene glycol, and mono- and polybutylene glycol, i.e., chains based on repeating units -CH₂CH₂O-, - CH₂CH₂CH₂O-, or -CH₂CH₂CH₂CH₂O-. The alkylene glycol portion can be monodisperse (having well-defined length/molecular weight) as well as polydisperse (having less well-defined length/average molecular weight). Monomeric alkylene glycol portions include chains with different end groups, such as -OCH₂CH₂O-, -OCH₂CH₂CH₂O- or -OCH₂CH₂CH₂CH₂O-.

The term "fatty acid" includes straight-chain or branched-chain aliphatic carboxylic acids having at least two carbon atoms and being saturated or unsaturated. Non-limiting examples of fatty acids include myristic acid, palmitic acid, stearic acid, and eicosanoic acid.

Here, the term "fatty diacid includes straight-chain or branched-chain aliphatic dicarboxylic acids having at least two carbon atoms and being saturated or unsaturated. Non-limiting examples of fatty diacid include adipic acid, suberic acid, azelaic acid, dodecanedioic acid, tetradecanedioic acid, hexadecanedioic acid, heptadecanedioic acid, octadecanedioic acid, eicosanedioic acid, docosanedioic acid, and tetracosanedioic acid.

In this document, the naming of GLP-1 compounds is based on the following principles: Names are assigned based on mutations and modifications (e.g., acylations) relative to native GLP-1 (7-37). The naming of acyl portions follows the IUPAC naming rules and is otherwise based on peptide naming conventions. For example, the following acyl portion could be named as :
It can be named, for example, "Eicosandiacyl-γGlu-OEG-OEG," " Eicosandiacyl-γGlu-2xOEG," or "Eicosandiacyl-gGlu-2xOEG," "19-carboxynonadecanoyl-yGlu-2xOEG," or "19-carboxynonadecanoyl-γGlu-OEG-OEG," where OEG represents the group - NH(CH₂)₂O(CH₂)₂OCH₂CO- (i.e., abbreviation for the group 2-[2-(2-aminoethoxy)ethoxy]acetyl), and γGlu (and gGlu) is the abbreviation for the L-isomer of amino acid γ-glutamate. Alternatively, the acyl portion can be named according to IUPAC naming conventions (OpenEye, IUPAC format). According to this naming convention, the above acyl portion is referred to by the following names: "[2-[2-[2-[2-[2-[2-[(4S)-4-carboxy-4-(19-carboxynonadecanoylamino) butanoyl] amino]ethoxy]ethoxy]acetyl]amino]ethoxy] ethoxy]acetyl]" or "[2-(2-[2-(2-[2-(2-[4-(19- carboxynonadecanoylamino)-4(S)-carboxybutyryl amino]ethoxy)ethoxy]acetylamino)ethoxy]ethoxy)acetyl]".

In this document, the term "delivery agent or N-(8-(2-hydroxybenzoyl)amino)caprylic acid (NAC) salt" refers to excipients capable of enhancing oral exposure of the GLP-1 compound.

In a specific example, the salt of N-(8-(2-hydroxybenzoyl)amino)caprylic acid (NAC) contains the anion N-(8-(2-hydroxybenzoyl)amino)caprylic acid anion. The structural formula of N-(8-(2-hydroxybenzoyl)amino)caprylic acid anion is shown in Formula (I).

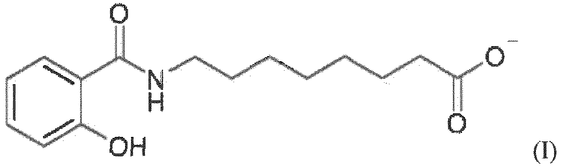

In another specific example, the salt of N-(8-(2-hydroxybenzoyl)amino)caprylic acid is selected from sodium salt. The salt of N-(8-(2-hydroxybenzoyl)amino)caprylic acid can be prepared using methods described, for example, in WO96/030036, WO00/046182, WO01/092206, or WO2008/028859.

In a specific example, the pharmaceutical composition includes at least one pharmaceutically acceptable excipient. The term "excipient" as used herein broadly refers to any component other than the active therapeutic ingredient. Excipients can be inert substances, inactive substances, and/or non-drug active substances. Excipients can be used for various purposes, such as carriers, solvents, binders, lubricants, flow enhancers, diluents, and/or for improving administration and/or absorption of the active substance. The formulation of the active drug ingredient with different excipients is known in the art, see, for example, Remington: The Science and Practice of Pharmacy (e.g., 19th edition, 1995, and any updated version).

In some examples, the specific values mentioned in this document regarding numbers or intervals can be understood as the exact value or approximately that value. In some examples, the term "about" refers to the mentioned value ±10%, so about 100 mM includes 100 mM ± 10 mM, about 10% includes 10% ± 1%, and so on.

In some examples, the concentrations of the components in the pharmaceutical composition are expressed as %(w/w), which refers to the mass percentage of the component relative to the total mass of the pharmaceutical composition. For example, 20% (w/w) polyvinyl ketone means that the mass of polyvinyl ketone is 20% of the total mass of the pharmaceutical composition.

The GLP-1 derivatives and analogs of the present invention have GLP-1 activity. Having GLP-1 activity refers to the ability to bind to GLP-1 receptors and initiate signaling pathways, resulting in insulinotropic effects or the ability of other physiological effects.

The term "bioavailability" refers to the fraction of an active pharmaceutical ingredient (API) in a formulation, such as the GLP-1 compounds herein, that accounts for the administered dose when it reaches the systemic circulation unchanged. By definition, the bioavailability is 100% when the API is administered intravenously. However, when administered by other routes (e.g., orally), the bioavailability is reduced (due to incomplete absorption and first-pass metabolism). Understanding bioavailability is important when calculating doses for non-intravenous routes of administration.

In this document, "efficacy" refers to the ability of a pharmaceutical composition to produce a certain action or effect (such as lowering blood glucose).

### Acronyms:

Na₂HPO₄: Disodium phosphate,
NaOH: Sodium hydroxide,
OEG: Amino acid residue-NH(CH₂)₂O(CH₂)₂OCH₂CO-,
OSu: N-hydroxysuccinimide ester-1-oxy-2,5-dioxopyrrolidine-1-yl ester,
OtBu: tert-Butyl ether,
HCl: Hydrochloric acid,
γGlu or gGlu: γ-L-glutamyl group,
NHS: N-hydroxysuccinimide,
DCC: Dicyclohexylcarbodiimide,
AEEA: 2-(2-(2-aminoethoxy)ethoxy)acetic acid,
OH: Hydroxide,
Gly: Glycine,
Arg: Arginine
TFA: Trifluoroacetic acid,
HbA1c: Glycated hemoglobin,
RD%: Relative deviation,

### Example 1

### Title compound:

### N-ε²⁶-[2-(2-[2-(2-[2-(2-[4-(19-carboxynonadecanoylamino)-4(S)-carboxybutanoylamino]ethoxy)ethoxy]acetylamino)ethoxy]ethoxy)acetyl][Gly8, Arg34]GLP-1-(7-37) peptide (Compound 1)

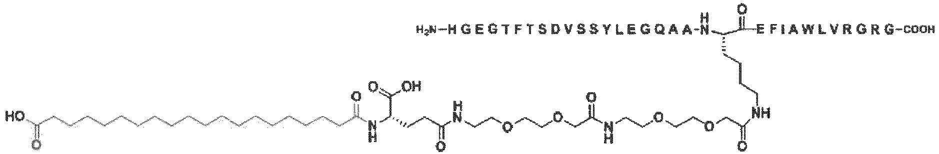

### 1.Preparation of N-ε²⁶-[2-(2-[2-(2-[2-(2-[4-(19-carboxynonadecanoylamino)-4(S)-carboxybutanoylamino]ethoxy)ethoxy]acetylamino)ethoxy]ethoxy)acetyl][Gly8, Arg34]GLP-1-(7-37) peptide

The [Gly8,Arg34]GLP-1-(7-37) peptide (5g, 1.48mmol) was prepared using general protein recombinant expression methods (specific methods can be found in Molecular Cloning: A Laboratory Manual (Fourth Edition), Michael R. Green, Cold Spring Harbor Press, 2012). The [Gly8,Arg34]GLP-1-(7-37) peptide (5g, 1.48mmol) was dissolved in a 100mM Na₂HPO₄ aqueous solution (150mL) and acetonitrile (100mL) was added. The pH was adjusted to pH 10-12.5 using 1N NaOH. The tert-butyl eicosanedioyl-γGlu(2xOEG-OSu)-OtBu (1.59g, 1.63mmol) was dissolved in acetonitrile (50mL) and slowly added to the [Gly8,Arg34]GLP-1-(7-37) peptide solution. The pH was maintained at 10-12.5. After 120 minutes, the reaction mixture was added to water (150mL) and the pH was adjusted to 5.0 using 1N HCl aqueous solution. The precipitate was separated by centrifugation and freeze-dried. The crude product was added to a mixture of trifluoroacetic acid (60mL) and dichloromethane (60mL) and stirred at room temperature for 30 minutes. The mixture was concentrated to approximately 30mL and poured into ice-cold n-heptane (300mL). The precipitated product was separated by filtration and washed twice with n-heptane. After vacuum drying, the product was purified by ion exchange chromatography (Ressource Q, 0.25%-1.25% ammonium acetate gradient in 42.5% ethanol, pH 7.5) and reversed-phase chromatography (acetonitrile, water, TFA). The purified fractions were combined, and the pH was adjusted to 5.2 using 1N HCl. The precipitate was separated and freeze-dried to obtain the title compound.
LC-MS (electrospray): m/z = 1028.79 [M+4H]4⁺

### 2. Preparation of Intermediate tert-Butvl eicosanediovl-γGlu-(2xOEG-OSu)-OtBu

### 2.1 tert-Butyl eicosanedioyl-OSu

Under nitrogen protection, Eicosanedic acid tert-butyl ester (20g, 50.17mmol) and NHS (5.77g, 50.17mmol) were mixed in dichloromethane (400mL) and triethylamine (13.95mL) was added. The resulting turbid mixture was stirred at room temperature, and then DCC (11.39g, 55.19mmol) was added and further stirred overnight. The mixture was filtered, and the filtrate was concentrated to almost dryness. The residue was mixed with cold water and ethyl acetate, stirred for 20 minutes, and then separated. The organic phase was washed with saturated brine, and the upper organic phase was dried over anhydrous sodium sulfate, filtered, and the filtrate was concentrated under vacuum to almost dryness. The residue was vacuum dried overnight to obtain tert-butyl eicosanedioyl-OSu with a yield of 24.12g (97% yield).
LC-MS (Scie × 100API): m/z = 496.36 (M+1)⁺

### 2.2 tert-Butyl eicosanedioyl-γGlu-OtBu

Tert-butyl eicosanedioy1-OSu (24.12g, 48.66mmol) was dissolved in dichloromethane (250mL) and stirred, followed by the addition of H-Glu-OtBu (10.88g, 53.53mmol) and triethylamine (12.49mL). Water (25mL) was added, and the mixture was heated to obtain a clear solution, which was stirred at room temperature for 4 hours. Then, a 10% aqueous citric acid solution (200mL) was added, and the mixture was partitioned. The lower organic phase was washed with saturated brine, and the organic phase was dried over anhydrous sodium sulfate. After filtration, the filtrate was concentrated under reduced pressure to almost dryness, followed by vacuum drying overnight. Tert-butyl eicosanedioyl-γGlu-OtBu was obtained with a yield of 27.27g (96% yield).
LC-MS (Scie × 100API): m/z = 584.44 (M+1)⁺

### 2.3 tert-Butyl eicosanedioyl-γGlu-(OSu)-OtBu

Under nitrogen protection, tert-butyl eicosanedioyl-γGlu-OtBu (27.27g, 46.71mmol) was dissolved in dichloromethane (300mL), and triethylamine (11.99mL) was added and stirred for 10 minutes. NHS (5.38g, 50.17mmol) was then added, followed by the addition of DCC (10.60g, 51.38mmol). The mixture was stirred overnight at room temperature. After filtration, the filtrate was concentrated to almost dryness, and the residue was mixed with cold water and ethyl acetate, stirred for 20 minutes, and then separated. The organic phase was washed with saturated brine, and the upper organic phase was dried over anhydrous sodium sulfate, filtered, and the filtrate was concentrated under reduced pressure to almost dryness. The residue was dissolved in methyl tert-butyl ether, stirred for 30 minutes, filtered under reduced pressure, and the filter cake was vacuum dried overnight to obtain tert-butyl eicosanedioyl-γGlu-(OSu)-OtBu with a yield of 25.76g (81% yield).
LC-MS (Scie × 100API): m/z = 681.46 (M+1)⁺

### 2.4 tert-Butyl eicosanedioyl-γGlu-(2xOEG-OH)-OtBu

Tert-butyl eicosanedioyl-γGlu-(OSu)-OtBu (25.76g, 37.83mmol) was dissolved in dichloromethane (250mL) and stirred, followed by the addition of 2xAEEA (11.66g, 37.83mmol) and triethylamine (9.71mL). Water (25mL) was added, and the mixture was heated to obtain a clear solution, which was stirred at room temperature for 4 hours. Then, a 10% aqueous citric acid solution (200mL) was added, and the mixture was partitioned. The lower organic phase was washed with saturated brine, and the organic phase was dried over anhydrous sodium sulfate. After filtration, the filtrate was concentrated under reduced pressure to almost dryness, followed by vacuum drying overnight. Tert-butyl eicosanedioyl-γGlu-(2xOEG-OH)-OtBu was obtained with a yield of 30.75g (93% yield).
LC-MS (Scie × 100API): m/z = 874.59 (M+1)

### 2.5 tert-Butyl eicosanedioyl-γGlu-(2xOEG-OSu)-OtBu

Under nitrogen protection, tert-butyl eicosanedioyl-γGlu-(2xOEG-OH)-OtBu (30.75g, 35.18mmol) was dissolved in dichloromethane (300mL), and triethylamine (9.03mL) was added and stirred for 10 minutes. NHS (4.05g, 35.18mmol) was then added, followed by the addition of DCC (7.98g, 38.70mmol). The mixture was stirred overnight at room temperature. After filtration, the filtrate was concentrated to almost dryness, and the residue was mixed with cold water and ethyl acetate, stirred for 20 minutes, and then separated. The organic phase was washed with saturated brine, and the upper organic phase was dried over anhydrous sodium sulfate, filtered, and the filtrate was concentrated under reduced pressure to almost dryness. The residue was vacuum dried overnight, and tert-butyl eicosanedioyl-γGlu-(2xOEG-OSu)-OtBu was obtained with a yield of 31.09g (91% yield).
LC-MS (Scie × 100API): m/z = 971.61 (M+1)⁺

### Example 2

Title compound: *N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37)peptide (Compound 2)

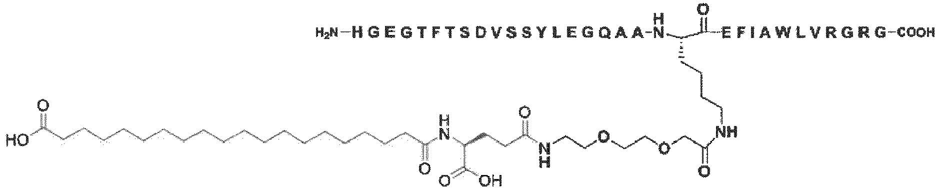

Preparation of *N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide was carried out following similar steps as described in Example 1, Part 1.
LC-MS (electrospray): m/z = 992.52 [M+4H]4⁺

The intermediate tert-butyl eicosanedioyl-γGlu-(OEG-OSu)-OtBu was prepared following similar steps as described in Example 1, Part 2.
LC-MS (Scie × 100API): m/z = 826.54 (M+1)⁺

### Example 3

Title compound: *N*-ε²⁶-(19-carboxynonadecanoylamino)-4(*S*)-carboxybutanoyl-[Gly8, Arg34]GLP-1-(7-37) peptide (Compound 3)

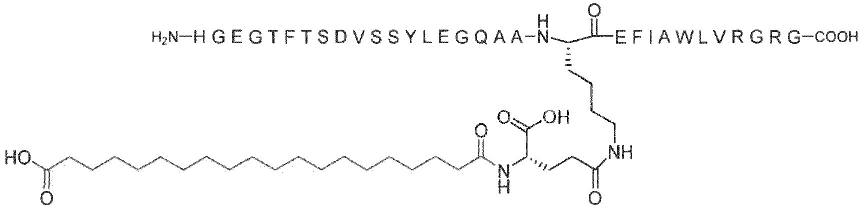

*N-*ε²⁶-(19-carboxynonadecanoylamino)-4(*S*)-carboxybutanoyl-[Gly8, Arg34]GLP-1-(7-37) peptide was prepared following similar steps as described in Example 1, Part 1.
LC-MS (electrospray): m/z = 956.25 [M+4H]4⁺

The intermediate tert-butyl eicosanedioyl-γGlu-(OSu)-OtBu was prepared following similar steps as described in Example 1, Part 2.
LC-MS (Scie × 100API): m/z = 681.46 (M+1)⁺

### Example 4

Title compound: *N*-ε²⁶-(19-carboxynonadecanoylamino)-4(*S*)-carboxybutanoyl-[Arg34]GLP-1-(7-37) peptide (Compound 4)

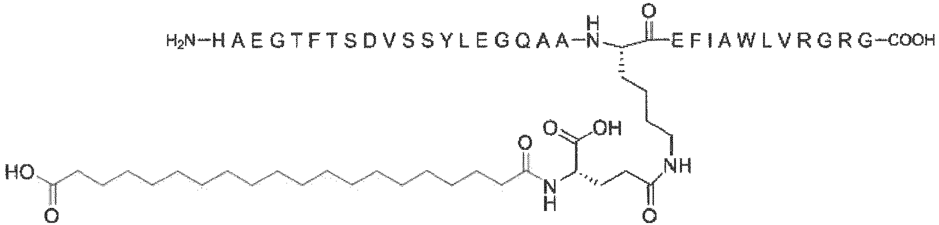

*N*-ε²⁶*-*(19-carboxynonadecanoylamino)-4(*S*)-carboxybutanoyl-[Arg34]GLP-1-(7-37) peptide was prepared following similar steps as described in Example 1, Part 1.
LC-MS (electrospray): m/z = 959.75 [M+4H]4⁺

The intermediate tert-butyl eicosanedioyl-γGlu-(OSu)-OtBuwas prepared following similar steps as described in Example 1, Part 2.
LC-MS (Scie × 100API): m/z = 681.46 (M+1)⁺

### Example 5:

Title compound: *N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide (Compound 5)

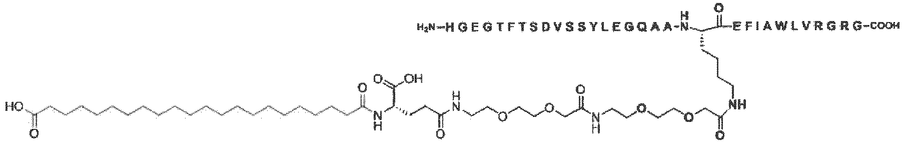

*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide was prepared following similar steps as described in Example 1, Part 1.
LC-MS (electrospray): m/z = 1035.80 [M+4H]4⁺

The intermediate tert-butyl docosanedioyl-γGlu-(2xOEG-OSu)-OtBu was prepared following similar steps as described in Example 1, Part 2.
LC-MS (Scie × 100API): m/z = 999.64 (M+1)⁺

### Example 6:

### Preparation of the Formulations

Tablets containing SNAC and GLP-1 compounds, as well as tablets containing only SNAC without the GLP-1 compound, were prepared according to the component contents shown in Table 1. The semaglutide compound used in this example was purchased from Gu Tuo Biotech Co., Ltd.

**Table 1. Tablet composition indicated as "per tablet"**

| Ingredients/Components | A | B | C | D | E | F | G | H | J |
|---|---|---|---|---|---|---|---|---|---|
| Compound 5(mg) | 7 | 10 | 7 | 7 | 7 | 20 | 0.7 | - | - |
| Semaglutide(mg) | - | - | - | - | - | - | - | 0.7 | - |
| SNAC(mg) | 350 | 350 | 300 | 150 | 400 | 350 | 30 | 30 | 30 |
| Polyvinylpyrrolidone K90(mg) | 8 | 8 | 8 | 8 | 8 | 8 | 0.8 | 0.8 | 0.8 |
| Microcrystalline cellulose(mg) | 68 | 68 | 68 | 68 | 68 | 68 | 6.8 | 6.8 | 6.8 |
| Magnesium stearate(mg) | 14 | 14 | 14 | 14 | 14 | 14 | 1.4 | 1.4 | 1.4 |
| Silica(mg) | 8 | 8 | 8 | 8 | 8 | 8 | 0.8 | 0.8 | 0.8 |
| Tablet weighting) | 455 | 458 | 405 | 255 | 505 | 468 | 40.5 | 40.5 | 39.8 |

According to the component contents in Table 1, the corresponding formulations were prepared using the following methods:
(1) Premix Step 1: SNAC, colloidal Silica and a portion of magnesium stearate were passed through a 40-mesh sieve together. The mixture was then added to a hopper blender and mixed at a speed of 20 rpm for 20 minutes to obtain Premix Powder 1.
(2) Premix Step 2: The GLP-1 compound was passed through a sieve along with microcrystalline cellulose and polyvinylpyrrolidone K90. The mixture was added to the above Premix Powder 1 and mixed at a speed of 20 rpm for 20 minutes to obtain Premix Powder 2.
(3) Granulation: Premix Powder 2 was placed in a dry granulator for one-step granulation. The horizontal screw feeder, roller speed of 2 rpm, roller pressure of 40 bar, feed screw speed of 40 rpm, and roller gap of 0.5-2 mm were used. The granules were sieved using a 1.2 mm mesh.
(4) Final Blend: The obtained granules were mixed with the remaining magnesium stearate in a hopper blender at a speed of 10 rpm for 5 minutes to obtain the final blend granules.
(5) Tablet Compression: The final blend granules were compressed into tablets, controlling the hardness and weight of the tablets, to obtain the tablet products.

Based on the component contents in Table 2, formulations containing SNAC and GLP-1 compound, as well as formulations containing only SNAC without the GLP-1 compound, were prepared. The solvent used for all formulations was purified water.

**Table 2.**

| Ingredients/Co mponents | I | II | III | IV | V | VI | VII | VIII | IX | X | XI | XII |
|---|---|---|---|---|---|---|---|---|---|---|---|---|
| Coumpound 5 (mg/ml) | 3 | 3 | 3 | 3 | - | - | 1 | - | - | 2 | - | - |
| Semaglutide (mg/ml) | - | - | - | - | 3 | - | - | 1 | - | - | 2 | - |
| SNAC (mg/ml) | 64.3 | 128. 5 | 149. 9 | 171. 4 | 128.5 | 171.4 | 42.8 | 42.8 | 42.8 | 85.7 | 85.7 | 85. 7 |

### Comparative Example 1

### Separate granulation process of the composition

**Table 3. Composition of Control Formulation A Tablets, expressed as "per tablet"**

| Component | | Content of components in control compound A(mg) |
|---|---|---|
| Granule 1 | SNAC | 300.0 |
| | Microcrystalline cellulose | 45.0 |
| | Magnesium stearate | 12 |
| | Colloidal silica | 8 |
| Granule 2 | Compound 5 | 7.0 |
| | Microcrystalline cellulose | 23.0 |
| | Polyvinylpyrrolidone K90 | 8.0 |
| Magnesium stearate | | 2.0 |
| Total | | 405 |

According to the content provided in Table 3, the following granulation process is used to prepare Control Formulation A through a separate granulation method:
Based on the quantities of the components in Granule 1, weigh SNAC, magnesium stearate, and colloidal silica, and place them in a blender. Mix for 20 minutes. Add microcrystalline cellulose to the blender and mix for another 20 minutes. The resulting mixture is granulated by using a dry granulator to obtain granule 1.

Based on the quantities of the components in Granule 2, weigh Compound 5, microcrystalline cellulose, and polyvinylpyrrolidone K90, and place them in the blender. Mix for 5 minutes. The resulting mixture is granulated by using a dry granulator to obtain granule 2.

Combine Granule 1, Granule 2, and magnesium stearate in the blender and mix for 5 minutes, then take the total mixed powder and compress it on a rotary tablet press.

### Example 7: Dissolution Experiment

According to the Chinese Pharmacopoeia 2020, 4th Part General Rules for Dissolution Test 0931, Method 2 (Paddle Method), the dissolution of Composition B, Composition C, Composition F, and the reference semaglutide tablets (RYBELSUS, a product of Novo Nordisk) in Example 6 are determined. The dissolution tests are conducted respectively by using 900 mL of dissolution media of hydrochloric acid-potassium chloride buffer (pH 2.0) containing 0.8% Tween 80, acetate buffer (pH 4.5) containing 1.0% Tween 80, phosphate buffer (pH 6.8) containing 0.1% Tween 80, and purified water. The tests are performed at 37°C with a paddle speed of 50 rpm. The results are shown in Tables 4-11.

**Table 4: Dissolution data of Scmaglutide in different dissolution media and similarity factor (F2) of dissolution curves between Semaglutide and SNAC in Semaglutide tablets.**

| Dissolution medium/time (min) | 0 | 15 | 30 | 45 | 60 | F2 factor |
|---|---|---|---|---|---|---|
| pH4.5+1.0% Tween | 0.0% | 49.3% | 82.4% | 95.3% | 98.7% | 67 |
| pH2.0+0.8% Tween | 0.0% | 56.3% | 91.0% | 99.8% | 102.0% | 71 |
| Purified water | 0.0% | 47.1% | 79.1% | 88.4% | 86.0% | 65 |

**Table 5: Dissolution data of SNAC in different dissolution media in Semaglutide tablets.**

| Dissolution medium/time (min) | 0 | 15 | 30 | 45 | 60 |
|---|---|---|---|---|---|
| pH4.5+1.0% Tween | 0.0% | 50.1% | 77.4% | 86.9% | 89.2% |
| pH2.0+0.8% Tween | 0.0% | 57.6% | 84.8% | 90.3% | 92.5% |
| Purified water | 0.0% | 50.4% | 73.8% | 81.2% | 84.5% |

**Table 6: Dissolution data of Compound 5 in different dissolution media and similarity factor (F2) of dissolution curves between Compound 5 and SNAC in Composition B.**

| Dissolution medium/time (min) | 0 | 15 | 30 | 45 | 60 | F2 factor |
|---|---|---|---|---|---|---|
| pH6.8+0.1% Tween | 0.0% | 68.5% | 96.0% | 96.8% | 96.8% | 84 |
| pH2.0+0.8% Tween | 0.0% | 71.5% | 101.6% | 102.4% | 102.8% | 82 |

**Table 7: Dissolution data of SNAC in different dissolution media in Composition B.**

| Dissolution medium/time(min) | 0 | 15 | 30 | 45 | 60 |
|---|---|---|---|---|---|
| pH6.8+0.1%Tween | 0.0% | 70.6% | 95.6% | 96.1% | 96.3% |
| pH2.0+0.8%Tween | 0.0% | 70.3% | 96.0% | 96.1% | 96.4% |

**Table 8: Dissolution data of Compound 5 in different dissolution media and similarity factor (F2) of dissolution curves between Compound 5 and SNAC in Composition C.**

| Dissolution medium/time(min) | 0 | 15 | 30 | 45 | 60 | F2 factor |
|---|---|---|---|---|---|---|
| pH6.8+0.1%Tween | 0.0% | 49.5% | 83.6% | 99.8% | 101.7% | 74 |
| pH4.5+1.0%Tween | 0.0% | 52.4% | 79.7% | 93.2% | 95.3% | 87 |
| pH2.0+0.8%Tween | 0.0% | 63.0% | 88.5% | 99.3% | 101.4% | 72 |
| Purified water | 0.0% | 45.2% | 77.1% | 92.6% | 96.0% | 61 |

**Table 9: Dissolution data of SNAC in different dissolution media in Composition C.**

| Dissolution medium/time(min) | 0 | 15 | 30 | 45 | 60 |
|---|---|---|---|---|---|
| pH6.8+0.1%Tween | 0.0% | 48.5% | 79.6% | 93.8% | 95.9% |
| pH4.5+1.0%Tween | 0.0% | 51.9% | 78.1% | 90.3% | 91.7% |
| pH2.0+0.8%Tween | 0.0% | 59.5% | 84.4% | 93.1% | 95.4% |
| Purified water | 0.0% | 54.0% | 78.9% | 88.8% | 91.0% |

**Table 10: Dissolution data of Compound 5 in different dissolution media and similarity factor (F2) of dissolution curves between Compound 5 and SNAC in Composition F.**

| Dissolution medium/time(min) | 0 | 15 | 30 | 45 | 60 | F2 factor |
|---|---|---|---|---|---|---|
| pH6.8+0.1%Tween | 0.0% | 67.4% | 95.0% | 95.6% | 95.8% | 73 |
| pH2.0+0.8%Tween | 0.0% | 70.0% | 97.6% | 97.3% | 97.1% | 92 |

**Table 11: Dissolution data of SNAC in different dissolution media in Composition F.**

| Dissolution medium/time(min) | 0 | 15 | 30 | 45 | 60 |
|---|---|---|---|---|---|
| pH6.8+0.1%Tween | 0.0% | 70.9% | 96.2% | 96.6% | 96.6% |
| pH2.0+0.8%Tween | 0.0% | 70.2% | 95.0% | 95.3% | 94.9% |

Based on the above experimental results, it can be concluded that the dissolution rate of the active ingredient and SNAC in the GLP-1 pharmaceutical compositions of the present invention is comparable or even closer to that of the semaglutide oral tablets, that is, it has a comparable or higher F2 factor, indicating that the pharmaceutical compositions of the present invention have better bioavailability.

### Example 8:

Following the experimental procedure described in Reference Example 7, the dissolution test was conducted on Compound A from Example 6 and the reference semaglutide tablets (product of Novo Nordisk) in a 500ml dissolution medium. The results are shown in Tables 12-15.

**Table 12: Dissolution data of semaglutide in different dissolution media and similarity factor (F2) of dissolution curves between semaglutide and SNAC in semaglutide tablets.**

| Dissolution medium/time(min) | Omin | 15min | 30min | 45min | 60min | F2 factor |
|---|---|---|---|---|---|---|
| pH2.0+0.8%Tween | 0.0% | 56.3% | 91.0% | 99.8% | 102.0% | 69 |
| pH4.5+1%Tween | 0.0% | 49.3% | 82.4% | 95.3% | 98.7% | 64 |
| pH6.8+0.1%Tween | 0.0% | 48.7% | 76.1% | 87.8% | 90.0% | 66 |

**Table 13: Dissolution Data of SNAC in Different Dissolution Media in Semaglutide Tablets**

| Dissolution medium/time(min) | 0min | 15min | 30min | 45min | 60min |
|---|---|---|---|---|---|
| pH2.0+0.8%Tween | 0.0% | 57.6% | 84.8% | 90.3% | 92.5% |
| pH4.5+1.0%Tween | 0.0% | 50.1% | 77.4% | 86.9% | 89.2% |
| pH6.8+0.1%Tween | 0.0% | 49.0% | 71.9% | 80.0% | 83.5% |

**Table 14: Dissolution Data of Compound 5 in Different Dissolution Media and Similarity Factor (F2) of Dissolution Curves between Compound 5 and SNAC in Composition A**

| Dissolution medium/time(min) | 0min | 15min | 30min * | 45min | 60min | F2 Factor |
|---|---|---|---|---|---|---|
| pH2.0+0.8%Tween | 0.0% | 63.0% | 88.5% | 99.3% | 101.4% | 72 |
| pH4.5+1.0%Tween | 0.0% | 52.4% | 79.7% | 93.2% | 95.3% | 85 |
| pH6.8+0.1 %Tween | 0.0% | 48.6% | 73.6% | 86.7% | 93.1 % | 69 |

**Table 15: Dissolution Data of SNAC in Different Dissolution Media in Composition A**

| Dissolution medium/time(min) | 0min | 15min | 30min | 45min | 60min |
|---|---|---|---|---|---|
| pH2.0+0.8%Tween | 0.0% | 59.5% | 84.4% | 93.1% | 95.4% |
| pH4.5+1%Tween | 0.0% | 51.9% | 78.1% | 90.3% | 91.7% |
| pH6.8+0.1%Tween | 0.0% | 45.0% | 69.7% | 82.3% | 88.5% |

Based on the above experimental results, it can be concluded that the dissolution rate of the active ingredient in the GLP-1 pharmaceutical compositions of the present invention is closer to that of SNAC, indicating a comparable or higher F2 factor. This suggests that the pharmaceutical compositions of the present invention have better bioavailability.

### Example 9:

The purpose of this experiment is to test the dissolution properties of GLP-1 pharmaceutical compositions of the present invention with different hardness levels.

Following similar procedures as in Example 7, the dissolution test was conducted on Composition B in Example 6 with different hardness levels. A 500 ml dissolution medium containing 0.8% Tween 80 in hydrochloric acid-potassium chloride buffer (pH 2.0) was used, and the dissolution experiment was performed at 37°C at a paddle speed of 50 rpm. The results are shown in Tables 16-17.

**Table 16: Dissolution data of Compound 5 in different dissolution media in Composition B.**

| **Hardness** | **0min** | **15min** | **30min** | **45min** | **60min** |
|---|---|---|---|---|---|
| 110N | 0.0% | 67.0% | 92.8% | 95.1% | 95.8% |
| 120N | 0.0% | 74.3% | 97.9% | 99.8% | 102.9% |
| 130N | 0.0% | 47.6% | 79.4% | 92.4% | 94.9% |

**Table 17 Dissolution data of SNAC in different dissolution media in composition B**

| **Hardness** | **0min** | **15min** | **30min** | **45min** | **60min** |
|---|---|---|---|---|---|
| 110N | 0.0% | 69.3% | 93.4% | 95.1% | 95.9% |
| 120N | 0.0% | 72.1% | 94.8% | 96.3% | 96.5% |
| 130N | 0.0% | 51.8% | 82.4% | 94.00% | 95.3% |

Based on the above experimental results, it can be concluded that the GLP-1 pharmaceutical compositions of the present invention exhibit good dissolution performance when the hardness is below 130N.

### Example 10:

The purpose of this experiment is to test the dissolution performance of GLP-1 pharmaceutical compositions of the present invention with active ingredient starting materials having different D90 particle sizes.

Following similar procedures as in Example 7, the dissolution test was conducted on Composition B in Example 6 for the active substance starting material with different D90 particle sizes. A 500 ml dissolution medium containing 0.8% Tween 80 in hydrochloric acid-potassium chloride buffer (pH 2.0) was used, and the dissolution experiment was performed at 37°C at a paddle speed of 50 rpm. The results are shown in Tables 18-19.

**Table 18: Dissolution data of Compound 5 in different dissolution media in Composition B.**

| **Particle size D90(µm)** | **0min** | **15min** | **30min** | **45min** | **60min** |
|---|---|---|---|---|---|
| 15 | 0.0% | 71.6% | 95.2% | 95.5% | 95.5% |
| 75 | 0.0% | 68.6% | 94.4% | 97.1% | 97.5% |
| 130 | 0.0% | 67.0% | 92.8% | 95.1% | 95.8% |

**Table 19: Dissolution data of SNAC in different dissolution media in Composition B.**

| **Particle size D90(µm)** | **0min** | **15min** | **30min** | **45min** | **60min** |
|---|---|---|---|---|---|
| 15 | 0.0% | 75.0% | 98.4% | 98.7% | 98.7% |
| 75 | 0.0% | 70.0% | 95.6% | 98.2% | 98.6% |
| 130 | 0.0% | 69.3% | 93.4% | 95.1% | 95.9% |

The results from the above experiments indicate that the GLP-1 pharmaceutical compositions of the present invention exhibit favorable dissolution performance when the particle size D90 of the active substance starting materials is between 15-130 um.

### Example 11: Pharmacodynamic Study in Normal Kunming (KM) Mice

The purpose of this study is to confirm the blood glucose and food intake control of the GLP-1 pharmaceutical compositions of the present invention.

Male Kunming (KM) mice (purchased from Weitong Lihua) were housed in a barrier environment in appropriately sized cages, with free access to standard food and purified water. Environmental conditions were maintained at a relative humidity of 40%-60% and a temperature of 22°C-26°C. After an adaptation period of 1-2 weeks, the mice were used for the experiment

On the day prior to the experiment, the mice were fasted, and basal blood glucose was assessed along with weighing the mice. Based on random blood glucose and weight, the mice were divided into treatment groups, control groups, or solvent groups (SNAC group). There were a total of 6 groups with 6 mice in each group. The mice received the following treatments: oral gavage suspension administration(10mL/kg). The treatment group received compositions I, II, III, and IV, the control group received composition V, and the solvent group received composition VI.

Oral gavage administration was conducted (10 mL/kg body weight), and blood glucose was measured at 1.5 hours after administration (considered as time 0). An intraperitoneal glucose tolerance test (ipGTT) was performed at 2 hours after administration. The steps were as follows: Blood was collected from the tail tip at specified time points to measure fasting blood glucose. Subsequently, a glucose solution (20% glucose, 10 ml/kg) was administered intraperitoneally, and blood glucose values were monitored at 0.5, 1, and 2 hours after glucose administration. The ipGTT experiment was conducted twice. Clean the tails of the mice with alcohol cotton balls. Blood samples were collected from the tail using a disposable blood collection needle, and blood glucose was measured using a glucose meter (Roche) and matching test strips. After the ipGTT experiment, the mice were given food intake. Food intake was monitored at 9 PM on the same day and 9 AM on the following day.

Dose-response curves of blood glucose over time after administration were plotted. In order to more intuitively and quantitatively illustrate the effect of GLP-1 compositon on blood glucose, for each individual dose-response curve, the area under the blood glucose-time curve (AUC) from 0 to the end of the monitoring period was calculated. A smaller AUC value indicates better glucose-lowering effect and better efficacy.

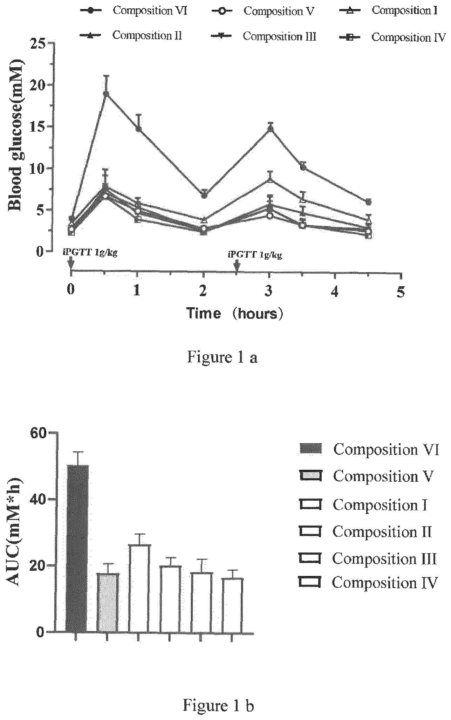

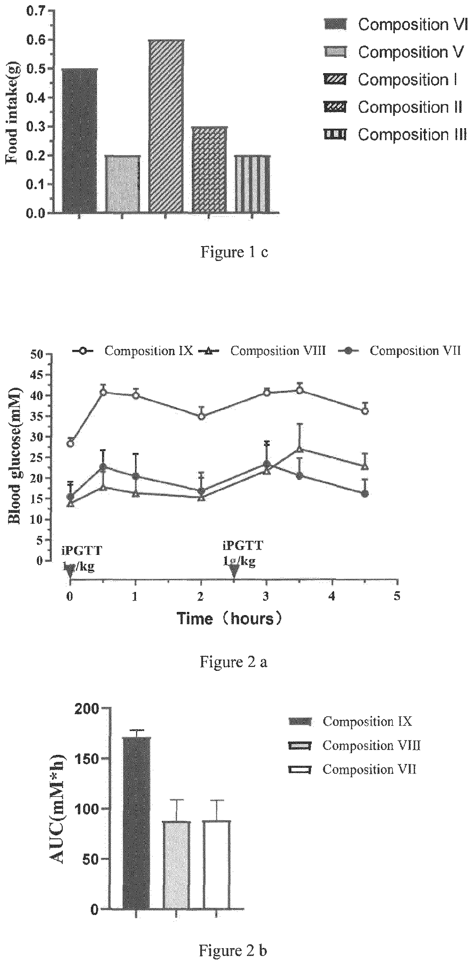

Figures 1a-1c demonstrate the glucose-lowering and appetite-inhibiting effects of the GLP-1 compositions of the present invention after oral gavage administration. Figures 1a and 1b show that compositions containing compound 5 and SNAC exhibit glucose-lowering effects in KM mice, and when the mass ratio of compound 5 to SNAC is 7:350 or 7:400, the glucose-lowering effect is comparable or even better than that of the composition containing semaglutide. Figure 1c shows that when the mass ratio of compound 5 to SNAC is 7:350, the appetite-inhibiting effect is equivalent to that of the same dose of semaglutide.

### Example 12: Pharmacodynamic Study in Type II Diabetic db/db Mice

The purpose of this study is to confirm the blood glucose control of the GLP-1 pharmaceutical compositions of the present invention in db/db mice.

Male db/db mice (Cavins) were housed in a barrier environment in appropriately sized cages, with free access to standard food and purified water. Environmental conditions were maintained at a relative humidity of 40%-60% and a temperature of 22°C-26°C. After an adaptation period of 1-2 weeks, the mice were used for the experiment.

On the day prior to the experiment, the mice were fasted, and basal blood glucose was assessed along with weighing the mice. Based on random blood glucose and weight, the diabetic mice were divided into treatment groups, control groups, or solvent groups (SNAC group). There were a total of 3 groups with 6 mice in each group. The mice received the following treatments: oral gavage suspension administration (10 mL/kg). The treatment group received composition VII, the control group received composition VIII, and the solvent group received composition IX.

Oral gavage administration was conducted (10 mL/kg body weight), and blood glucose was measured at 0.5 hours after administration (considered as time 0). An ipGTT experiment (10% glucose, 10mL/kg)was performed at 1 hour after administration with reference to example 11. Blood glucose values were monitored at 0.5, 1, and 2 hours after glucose administration, and two consecutive ipGTT experiments were conducted.

Clean the tails of the mice with alcohol cotton balls. Blood samples were collected from the tail using a disposable blood collection needle, and blood glucose was measured using a glucose meter (Roche) and matching test strips.

Dose-response curves of blood glucose over time after administration were plotted. In order to more intuitively and quantitatively illustrate the effect of GLP-1 compositon on blood glucose, for each individual dose-response curve, the area under the blood glucose-time curve (AUC) from 0 to the end of the monitoring period was calculated. A smaller AUC value indicates better glucose-lowering effect and better efficacy.

As shown in Figures 2a-2b, the GLP-1 compositions of the present invention exhibit unexpectedly increased glucose-lowering effects in db/db mice after administration. This further confirms that the glucose-lowering effect of the compositions containing compound 5 and SNAC of the present invention in db/db mice after administration is substantially consistent with the glucose-lowering effect after oral gavage of an equivalent dose of semaglutide.

### Example 13: Pharmacodynamic Study in SD Rats

The purpose of this study is to confirm the blood glucose control of the GLP-1 pharmaceutical compositions of the present invention.

Male SD rats (Weitong Lihua) were housed in a barrier environment in appropriately sized cages, with free access to standard food and purified water. Environmental conditions were maintained at a relative humidity of 40%-60% and a temperature of 22°C-26°C. After an adaptation period of 1-2 weeks, the rats were used for the experiment

On the day prior to the experiment, the rats were fasted for 16 hours, and basal blood glucose was assessed along with weighing the rats. Based on random blood glucose and weight, the rats were divided into treatment groups, control groups, or solvent groups (SNAC group). There were a total of 3 groups with 6 rats in each group. The rats received the following treatments: oral gavage tablet administration (3 tablets/rat, 3 mL water). The treatment group received composition G, the control group received composition H, and the solvent group received composition J.

Oral gavage tablet administration was conducted, 3 tables/rat, and ipGTT was performed 2 hours after administration with reference to Example 11 (20% glucose, 10mL/kg). Blood glucose values were monitored at 0.5, 1, and 2 hours after glucose administration.

Clean the tails of the mice with alcohol cotton balls. Blood samples were collected from the tail using a disposable blood collection needle, and blood glucose was measured using a glucose meter (Roche) and matching test strips.

Dose-response curves of blood glucose over time after administration were plotted. In order to more intuitively and quantitatively illustrate the effect of GLP-1 compositon on blood glucose, for each individual dose-response curve, the area under the blood glucose-time curve (AUC) from 0 to the end of the monitoring period was calculated. A smaller AUC value indicates better glucose-lowering effect and better efficacy.

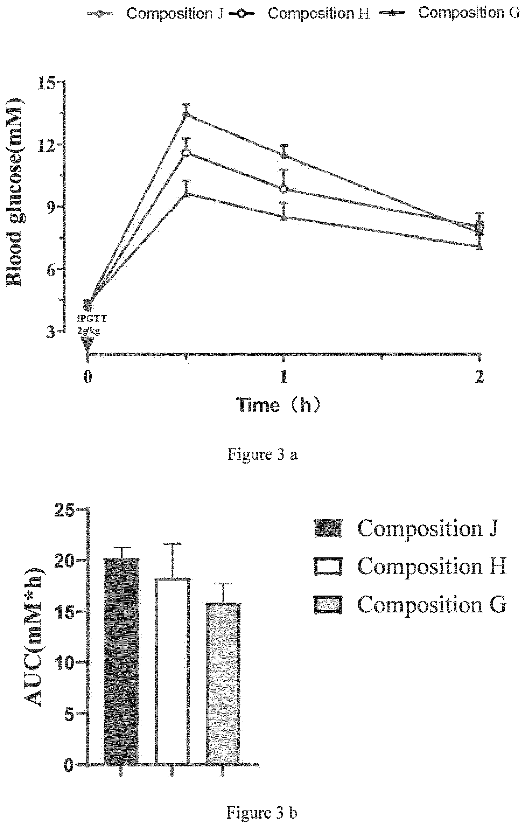

Figures 3a-3b demonstrate that the GLP-1 compositions of the present invention exhibit unexpectedly superior glucose-lowering effects in SD rats after oral gavage administration, compared to semaglutide composition.

### Example 14: Pharmacodynamic Study in ICR Mice

The purpose of this study is to confirm the blood glucose control of the GLP-1 pharmaceutical compositions of the present invention in ICR mice.

ICR mice were housed in a barrier environment in appropriately sized cages, with free access to standard food and purified water. Environmental conditions were maintained at a relative humidity of 40%-60% and a temperature of 22°C-26°C. After an adaptation period of 1-2 weeks, the mice were used for the experiment.

On the day prior to the experiment, the mice were fasted for 16 hours, and basal blood glucose was assessed along with weighing the mice. Based on random blood glucose and weight, the mice were divided into treatment groups, control groups, or solvent groups (SNAC group). There were a total of 3 groups with 6 mice in each group. The mice received the following treatments: oral gavage suspension administration (10 mL/kg). The treatment group received composition X, the control group received composition XI, and the solvent group received composition XII.

Oral gavage administration was conducted, and blood glucose was measured at 1.5 hours after administration as time 0. An intraperitoneal glucose tolerance test (ipGTT) was performed 2 hours after administration with reference to Example 11 (10% glucose, 10mL/kg). Blood glucose values were monitored before glucose administration, and at 0.5, 1, and 2 hours after glucose administration. Clean the tails of the mice with alcohol cotton balls. Blood samples were collected from the tail using a disposable blood collection needle, and blood glucose was measured using a glucose meter (Roche) and matching test strips. ipGTT experiments were repeated on the second and third days.

Dose-response curves of blood glucose over time after administration were plotted. In order to more intuitively and quantitatively illustrate the effect of GLP-1 compositon on blood glucose, for each individual dose-response curve, the area under the blood glucose-time curve (AUC) from 0 to the end of the monitoring period was calculated. A smaller AUC value indicates better glucose-lowering effect and better efficacy.

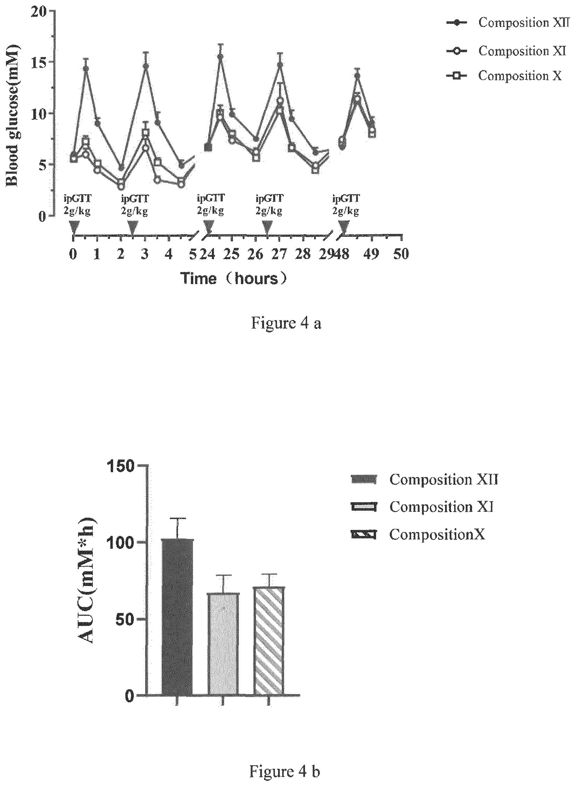

Figures 4a-4b show that the compositions of compound 5 and SNAC in the present invention exhibit glucose-lowering effects in ICR mice, and the glucose-lowering effect is comparable to that of compositions containing semaglutide.

### Example 15: Pharmacokinetics

The purpose of this example is to illustrate the *in vivo* pharmacokinetic properties of the pharmaceutical compositions of the present invention.

### Pharmacokinetics in SD Rats

A total of 25 male SD rats were divided into groups of 5 each, categorized as Composition B Low Dose Group (10 mg/kg), Composition B Medium Dose Group (15 mg/kg), and Composition B High Dose Group (20 mg/kg). The rats received a single oral dose via gastric gavage. Prior to dosing, the rats underwent a fasting period of approximately 16 hours. Blood samples were collected at various time points: pre-dose (0 min), 15 min, 30 min, 1 hr, 2 hr, 3 hr, 5 hr, 8 hr, 12 hr, 24 hr, 48 hr, and 72 hr post-dosing for the determination of blood drug concentration. Pharmacokinetic parameters such as Cₘₐₓ, Tₘₐₓ, T_{1/2}. AUC₀₋ₜ and MRT were calculated using the non-compartmental model with WinNonLin v6.4 software. The experimental results are presented in Table 20.

**Table 20: Pharmacokinetic Parameters After Oral Gavage Administration of Compound B to SD Rats**

| Dose(mg/kg) | Cₘₐₓ(ng/ml) | Tₘₐₓ(hr) | T_{1/2}(hr) | AUC₀₋ₜ (hr*ng/ml) | MRT(hr) |
|---|---|---|---|---|---|
| 10 | 123.00 | 0.5 | 8.63 | 801.39 | 10.21 |
| 15 | 51.90 | 2.5 | 9.94 | 571.80 | 13.42 |
| 20 | 103.65 | 2.0 | 9.81 | 1330.48 | 13.01 |

| | | | | | |
|---|---|---|---|---|---|
| Cₘₐₓ = Maximum measured plasma concentration Tₘₐₓ = Time at which maximum measured plasma concentration occurs T_{1/2} = Terminal elimination half-life AUC₀₋ₜ = Area under the glucose concentration-time curve from 0 to t MRT = Mean residence time | | | | | |

It can be seen from the above experiment that in SD rats, the composition of Compound 5 and SNAC of the present invention exhibits a long half-life and long mean residence time.

### Example 16: Uniformity of Granules Experiment

The purpose of this experiment is to demonstrate the uniformity of the compositions obtained using different preparation methods in the present invention.

Composition C was prepared using the one-step granulation process described in Example 6, while the control compound A was prepared using the separate granulation process described in comparative Example 1. After the mixing machines stopped rotating, multiple samples were taken at three horizontal positions (top, middle, and bottom) on the materials. The number of samples taken at each point was not less than 6, with a sampling amount at each point of 1-3 times the weight of a single tablet. After sampling, the content was analyzed using high-performance liquid chromatography (HPLC) according to the following steps:
A Phenomenex Luna C8 (4.6*50mm) column was used with a column temperature of 35°C and a sample chamber temperature of 5°C. The elution was performed at a flow rate of 1.0 ml/min. The eluent consisted of the following components:
Phase A: 0.1% trifluoroacetic acid aqueous solution
Phase B: 0.1% trifluoroacetic acid acetonitrile solution

The gradient was as follows: linear change of 70%/30% A/B to 35%/65% A/B from 0 to 8 minutes, linear change to 70%/30% A/B from 8-8.01 minutes, and a steady gradient of 70%/30% A/B from 8.01 to 13 minutes.

Detection wavelengths were set at 214 nm/235 nm, flow rate at 1.0 ml/min, and injection volume at 10 µl. Table 21 shows the uniformity of the total mixed granules obtained from the separate granulation and one-step granulation samples.

**Table 21**

| Sample | Mean purity of Compound 5 % | RSD% |
|---|---|---|
| Control composition A | 95.70 | 8.01 |
| Composition C | 101.4 | 2.82 |

Based on the above experimental results, it can be concluded that the sample granules of Compound C obtained through the one-step granulation process exhibit higher uniformity than that of the control compound A obtained through separate granulation.

### Example 17:

The chemical stability of the formulation in this example is represented by the changes in the quantity of related substances measured after storage for 1 month and 2 months under conditions of 30°C/65%RH.

### Measurement of Related Substances:

The content of related impurities was determined using high-performance liquid chromatography (HPLC) on a Waters Kromasil 100-3.5-C8 (4.6*250mm) column. The column temperature was set at 35°C, and the sample chamber temperature was set at 5°C. The elution was performed at a flow rate of 1.0 ml/min. The eluent consisted of the following components:
Phase A: 90 mM potassium dihydrogen phosphate and 10% acetonitrile (v/v), pH 2.4
Phase B: 75% acetonitrile (v/v)

The gradient was as follows: linear change of 750%/25% A/B to 55%/45% A/B from 0 to 5 minutes, linear change from 5 to 12 minutes to 50%/50% A/B, linear change from 12 to 42 minutes to 40%/60% A/B, linear change from 42 to 60 minutes to 100%/900% A/B, linear change from 60 to 61 minutes to 75%/25% A/B, and steady gradient of 75%/25% A/B from 61 to 70 minutes.

Detection wavelength was set at 214 nm, flow rate at 1.0 ml/min, and injection volume at 15µl. Table 22 shows the changes in the quantity of related substances relative to day 0 after storage for 1 month and 2 months at 30°C.

**Table 22**

| Storage temperature | Time | Total impurities % | Maximum individual impurity % | Purity of compound 5 % |
|---|---|---|---|---|
| 30°C/65%RH | 0 | 3.5 | 0.73 | 96.5 |
| | 1 month | 3.15 | 0.76 | 96.85 |
| | 2month | 3.33 | 1.03 | 96.67 |
| | 3month | 4.81 | 0.93 | 95.19 |
| 25°C/60%RH | 15month | 3.86 | 0.97 | 96.14 |

Based on the above table, it can be observed that the quantity of related substances in the GLP-1 pharmaceutical composition of the present invention increases very slowly over time, indicating excellent chemical stability of the above pharmaceutical composition.

### Example 18:

### Long-term Pharmacodynamic study in type 2 diabetes Kkay mice

The purpose of this study is to confirm the hypoglycemic effects of the GLP-1 compounds in the GLP-1 pharmaceutical composition of the present invention in type 2 diabetes Kkay mice.

Compound 5 from Example 5 and Compound 2 from Example 2 were tested on type 2 diabetes Kkay mice. The mice were administered different doses of Compound 5 and Compound 2, at 100 and 300 µg/kg, respectively. The effects of the GLP-1 compounds in the GLP-1 pharmaceutical composition of the present invention on reducing blood glucose levels and HbAlc were measured.

Male Kkay mice, 12-14 weeks old, were housed in suitable cages under barrier conditions with free access to standard food and purified water. The environmental conditions were controlled at a relative humidity of 40%-60% and a temperature of 22°C-24°C. After an adaptation period of 1-2 weeks, the mice were used for the experiment.

Basal blood glucose levels were assessed on the day of the experiment, and the mice were weighed. Based on random blood glucose and body weight, the diabetic mice were divided into a solvent group or a treatment group. The mice in the treatment group received subcutaneous injections of the GLP-1 compounds at doses of 100 and 300 µg/kg. The solvent consisted of propylene glycol (14 mg/ml), phenol (5.5 mg/ml), and disodium hydrogen phosphate (1.133 mg/ml) at pH 7.4.

Subcutaneous administration was performed at the nape of the neck (50µl/10g body weight). The GLP-1 compound in the GLP-1 pharmaceutical composition of the present invention or the solvent were administered at approximately 10:00 am (time 0), once every 2 days for a total of 16 doses. Blood glucose levels in the mice were evaluated at 3 hours, 6 hours, 1 day, and 2 days after the initial administration. HbAlc was measured 48 hours after the final administration using EDTA anticoagulant.

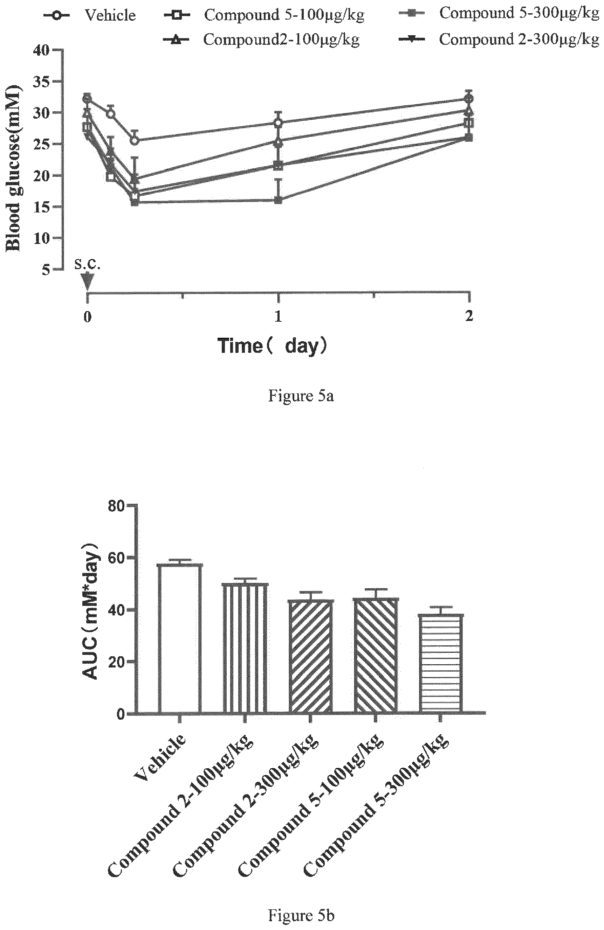

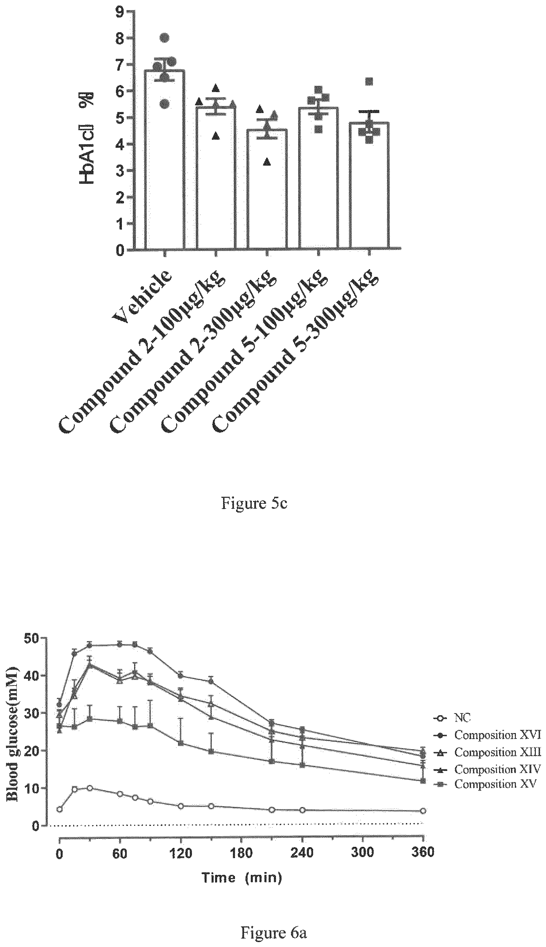

Figures 5a-5b show the unexpected increase in hypoglycemic efficacy of the GLP-1 derivatives used in the pharmaceutical composition of the present invention. Title Compounds from Example 5 and Example 2 exhibited significant hypoglycemic effects in Kkay mice. Figure 5c demonstrates the significant reduction in HbA1c observed in type 2 diabetes Kkay mice treated with title Compounds from Example 5 and Example 2.

### Example 19:

### Preparation of the compositions

According to the content of each component shown in Table 23, the tablet containing SNAC and the GLP-1 compound of the present invention was prepared by reference to the preparation method described in Example 6.

**Table 23: Composition of the tablets, expressed as "per tablet"**

| Components | K | L | M | N | O | P | Q | R | S |
|---|---|---|---|---|---|---|---|---|---|
| Compound 5(mg) | 15 | 80 | 10 | 20 | - | 3 | - | 40 | 60 |
| Semaglutide(mg) | - | - | - | - | 7 | - | - | - | - |
| SNAC(mg) | 300 | 300 | 300 | 300 | 300 | 18 | 18 | 300 | 300 |
| Polyvinylpyrrolidone K90(mg) | 8 | 8 | 8 | 8 | 8 | 0.5 | 0.5 | 8 | 8 |
| Microcrystalline cellulose(mg) | 68 | 68 | 68 | 68 | 68 | 0.8 | 0.8 | 68 | 68 |
| Magnesium stearate(mg) | 14 | 14 | 14 | 14 | 14 | 4.1 | 4.1 | 14 | 14 |
| Silica (mg) | 8 | 8 | 8 | 8 | 8 | 0.5 | 0.5 | 8 | 8 |
| Tablet weight(mg) | 413 | 478 | 408 | 418 | 405 | 26.9 | 23.9 | 438 | 458 |

Based on the component contents shown in Table 24, compositions containing SNAC and the GLP-1 compound of the present invention were prepared, as well as compositions containing only SNAC without the GLP-1 compound. The solvent for all formulations was purified water, and they were in the form of suspensions.

**Table 24**

| Ingredients/Components | XIII | XIV | XV | XVI |
|---|---|---|---|---|
| Compound 5(mg/ml) | 0.5 | 1.5 | 4.5 | 0 |
| SNAC(mg/ml) | 30 | 30 | 30 | 30 |

### Example 20

With reference to the experimental procedure described in Example 7, the dissolution of compounds K and L in Example 19 was determined. Phosphate buffer solution (pH 6.8) containing 0.1% Tween was used as the dissolution medium, and the dissolution experiments were conducted at 37°C with a paddle speed of 70 rpm. The results are shown in Tables 25 and 26.

**Table 25: Dissolution data of compound 5 in composition K and L**

| Sample | 0min | 10min | 15min | 20min | 30min |
|---|---|---|---|---|---|
| Composition L | 0.0% | 46.7% | 64.7% | 78.9% | 93.7% |
| Composition K | 0.0% | 48.3% | 67.3% | 82.1% | 96.5% |

**Table 26: Dissolution data of SNAC in composition K and L and similarity factor (F2) of the dissolution curves between SNAC and compound 5.**

| Sample | 0min | 10min | 15min | 20min | 30min | F2 |
|---|---|---|---|---|---|---|
| Composition L | 0.0% | 49.3% | 67.2% | 80.8% | 94.3% | 82.0 |
| Composition K | 0.0% | 48.1% | 65.8% | 79.1 % | 91.2% | 74.1 |

Based on the above experimental results, it can be observed that the dissolution rate of the active substance in the GLP-1 pharmaceutical composition of the present invention is similar to that of SNAC, indicating a higher F2 factor. This suggests that the pharmaceutical composition of the present invention has excellent bioavailability.

### Example 21

### Pharmacodynamic Study in Type 2 Diabetic db/db Mice

The purpose of this study was to confirm the blood glucose control effect of the GLP-1 pharmaceutical composition of the present invention in db/db mice.

Db/db mice (half male and half female) and m/m mice (half male and half female) were housed in barrier facilities in suitable cages with access to standard food and purified water. The environmental conditions were controlled at a relative humidity of 40%-60% and a temperature of 22°C-26°C. After an adaptation period of 1-2 weeks, the mice were used for the experiment.

Fasting was performed the day before the experiment, and basal blood glucose levels were evaluated before the start of the experiment. The mice were weighed. The m/m mice served as the normal control group, and the db/db mice were randomly assigned to the treatment group or the solvent group (SNAC group). There were a total of 5 groups, with 8 mice in each group. The treatment group and the solvent group received the following treatments: oral gavage with a suspension (10ml/kg). The treatment group received compositions VIII, XIV, and XV of the present invention, while the solvent group received composition XVI.

Oral gavage was performed (1ml/kg body weight), and blood glucose was measured at 0.5 hours after administration. At 1 hour after administration, an ipGTT test (10% glucose, 1g/kg) was performed according to Example 11. The blood glucose levels were monitored at 15 minutes, 30 minutes, 60 minutes, 75 minutes, 90 minutes, 120 minutes, 150 minutes, 210 minutes, 240 minutes, and 360 minutes after glucose administration.

The tail was cleaned with an alcohol swab, and blood drops were collected using a disposable blood collection needle. Blood glucose levels were measured using a glucose meter (Roche) and matching test strips.

Dose-response curves of blood glucose over time were plotted. To provide a more intuitive and quantitative representation of the effect of the GLP-1 compounds of the present invention on blood glucose, the area under the blood glucose-time curve (AUC) from 0 to the end of monitoring was calculated for each individual dose-response curve. A smaller AUC value indicates better glucose-lowering effect and better efficacy.

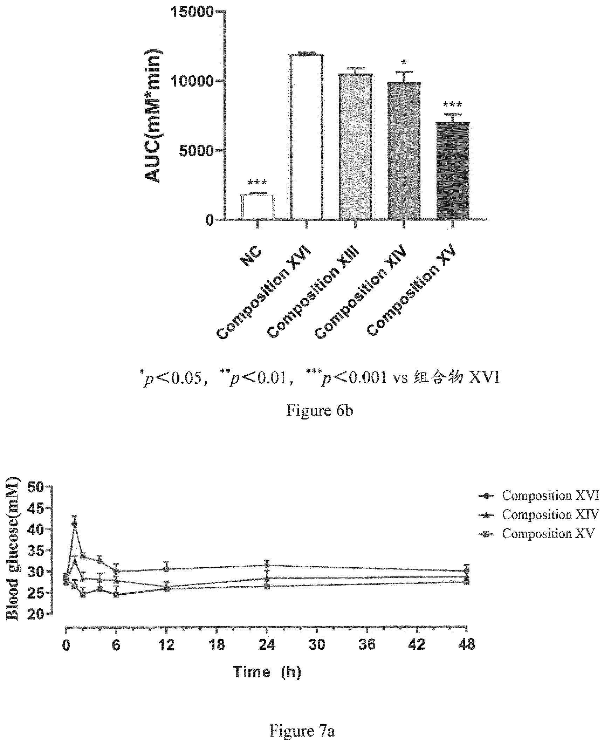

As shown in Figures 6a and 6b, the GLP-1 composition of the present invention exhibited an unexpected glucose-lowering effect after administration. The composition of title Compounds of Example 5 and SNAC showed significant glucose-lowering effects in db/db mice, and improved their glucose tolerance in a dose-dependent manner.

### Example 22

### Pharmacodynamic Study in Type 2 Diabetic db/db Mice

The purpose of this study was to confirm the control of blood glucose and food intake of the GLP-1 pharmaceutical composition of the present invention in db/db mice.

A similar experimental procedure as described in Example 21 was conducted to study the pharmacodynamic effects in type 2 diabetic db/db mice. The db/db mice were assigned to the treatment group or the solvent group (SNAC group) based on random blood glucose and weight. There were a total of 3 groups, with 8 mice in each group. The treatment group and the solvent group received the following treatments: oral gavage with a suspension (10ml/kg). The treatment group received composition XIV and XV of the present invention, while the solvent group received composition XVI.

Oral gavage was performed (10ml/kg body weight), and blood glucose levels of mice were monitored at 1 hour, 2 hours, 4 hours, 6 hours, 12 hours, 24 hours, and 48 hours after administration. Meanwhile, immediate post-administration feeding was resumed, and the mice's body weight and food intake were recorded at 0 minutes (before administration), 12 hours, 24 hours, 48 hours, and 72 hours after administration.

The tail was cleaned with an alcohol swab, and blood drops were collected using a disposable blood collection needle. Blood glucose levels were measured using a glucose meter (Roche) and matching test strips.

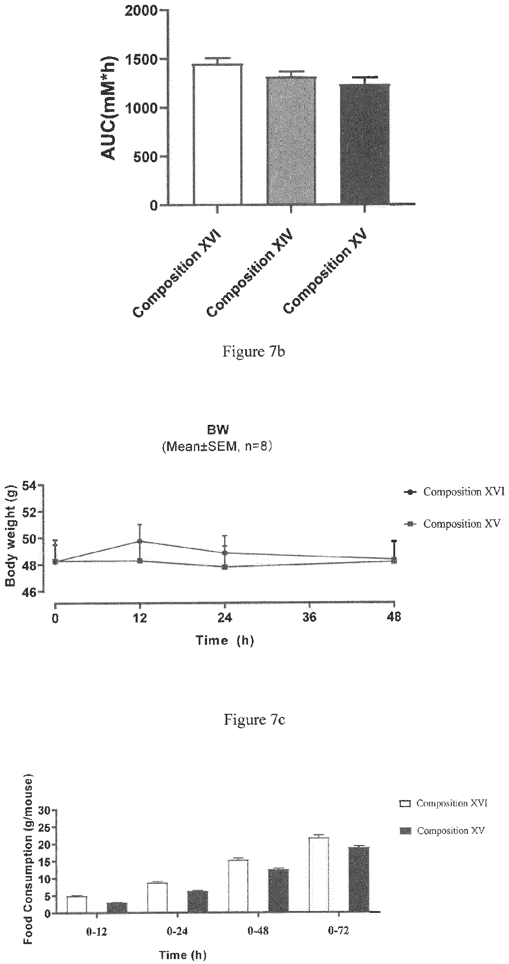

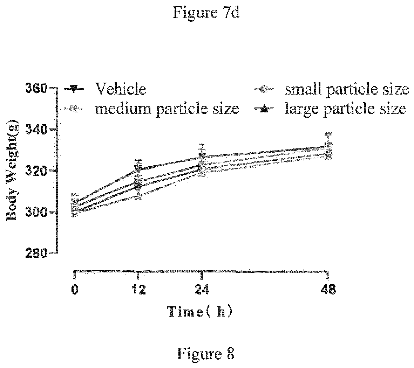

As shown in Figures 7a to 7d, the GLP-1 compositions of the present invention exhibited an unexpected glucose-lowering effect, weight loss effect, and appetite suppression effect after administration. Figures 7a and 7b demonstrated significant glucose-lowering effects of the composition of title compound of Example 5 and SNAC in db/db mice treated, while Figures 7c and 7d showed the weight-reducing and food intake-controlling effects of the composition of title compound of Example 5 and SNAC of the present invention in db/db mice.

### Example 23

The purpose of this example is to illustrate the pharmacokinetic properties *in vivo* of the present invention's compounds.

### Pharmacokinetics in Cynomolgus Monkeys

Nine cynomolgus monkeys (3 groups of 3 monkeys each, 2 males and 1 female in each group) were orally gavage administered composition M (low dose of compound 5), N (high dose of compound 5), and O (semaglutide, control group) for 7 consecutive days, once daily with one tablet. On the 7th day, blood samples were collected at 0 minutes (before administration), 15 minutes, 30 minutes, 1 hour, 2 hours, 4 hours, 8 hours, 12 hours, 24 hours after administration to measure the blood drug concentration. The pharmacokinetic parameters Cₘₐₓ, Tₘₐₓ, T_{1/2}, AUCot, MRT were calculated using the non-compartmental model of WinNonLin v6.4 software. The results are shown in Table 27.

**Table 27: Pharmacokinetic parameters after oral gavage administration in cynomolgus monkeys**

| Group | Cₘₐₓ(ng/ml) | Tₘₐₓ(hr) | T_{1/2}(hr) | AUC₀₋ₜ (hr*ng/ml) | MRT(hr) |
|---|---|---|---|---|---|
| Low Dose group | 328 | 1.67 | 29.8 | 5610 | 10.6 |
| High Dose group | 1140 | 4.67 | 37.4 | 16700 | 10.4 |
| Control group | 544 | 2.67 | 28.8 | 9400 | 10.4 |

Based on the above experimental results, it can be observed that in cynomolgus monkeys, the oral formulation of compound 5 of the present invention exhibits a comparable or even longer half-life compared to that of the control group treated with semaglutide oral formulation, and has a comparable average residence time.

### Example 24

### Pharmacodynamic and Pharmacokinetic Study in SD Rats

The purpose of this study was to confirm the weight control and pharmacokinetic properties of the GLP-1 pharmaceutical composition of the present invention in SD rats.

Male SD rats (Wei Tong Li Hua) were housed in barrier facilities in suitable cages with free access to standard food and purified water. The environmental conditions were controlled at a relative humidity of 40%-60% and a temperature of 22°C-26°C. After an adaptation period of 1-2 weeks, the rats were used for the experiment.

Fasting was performed the day before the experiment, and basal blood glucose levels were evaluated before the start of the experiment. The rats were weighed. The SD rats were assigned to the treatment group or the solvent group (SNAC group) according to random blood glucose and weight. There were a total of 4 groups, with 8 rats in each group. The treatment group and the solvent group received the following treatments: oral gavage with tablets (3 tablets/rat, 3 ml water). The treatment group received composition P with particle sizes of 15.7 µm (small particle group), 51.6 µm (medium particle group), and 134 µm (large particle group), while the solvent group received composition Q.

Oral gavage was performed with 3 tablets/rat. Body weight was measured at day 0 (before administration), 12 hours, 24 hours, and 48 hours after administration. Blood samples were collected at 0 hours (day -1), 15 minutes, 30 minutes, 1 hour, 2 hours, 3 hours, 8 hours, 24 hours, and 48 hours after administration to measure the blood drug concentration. The non-compartmental model of WinNonLin v6.4 software was used to calculate the pharmacokinetic parameters Cₘₐₓ, Tₘₐₓ, T_{1/2}, AUCo-t, MRT. The experimental results are shown in Table 28.

**Table 28: Pharmacokinetic parameters after oral gavage in SD rats**

| Groups | Cₘₐₓ(ng/ml) | Tₘₐₓ(hr) | T_{1/2}(hr) | AUC₀₋ₜ (hr*ng/ml) | MRT(hr) |
|---|---|---|---|---|---|
| Small Particle group | 82 | 0.938 | 10 | 786 | 13.5 |
| Medium Particle group | 65.4 | 1.13 | 9.23 | 691 | 12.1 |
| Big Particle group | 58 | 1.19 | 8.18 | 516 | 10.9 |

As shown in Figure 8, the oral formulation of compound 5 of the present invention exhibits unexpected weight loss effects within the particle size range mentioned above.

Furthermore, in SD rats, the oral formulation of compound 5 of the present invention demonstrates longer half-life, higher systemic exposure, and extended average residence time within the particle size range mentioned above.

### Sequence list

SEQ ID NO.1:
   GLP-1-(7-37) peptide 
His Ala Glu Gly Thr Phe Thr Ser Asp Val Ser Ser Tyr Leu Glu Gly Gln Ala Ala Lys Glu
Phe Ile Ala Trp Leu Val Lys Gly Arg Gly
SEQ ID NO.2:
   [Gly8, Arg34]OLP-1-(7-37) peptide 
His Gly Glu Gly Thr Phe Thr Ser Asp Val Ser Ser Tyr Leu Glu Gly Gln Ala Ala Lys Glu
Phe Ile Ala Trp Leu Val Arg Gly Arg Gly
SEQ ID NO.3:
   [Arg34]GLP-1-(7-37) peptide

His Ala Glu Gly Thr Phe Thr Ser Asp Val Ser Ser Tyr Leu Glu Gly Gln Ala Ala Lys Glu
Phe Ile Ala Trp Leu Val Arg Gly Arg Gly

## Claims

1. A pharmaceutical composition comprising a GLP-1 compound and a salt of N-(8-(2-hydroxybenzoyl)amino)caprylic acid (NAC), wherein the GLP-1 compound is a compound of formula (B) or a pharmaceutically acceptable salt, amide, or ester thereof:
[Acy-(L1)r(L2)_{q}]-G1 (B),
wherein G1 is a GLP-1 analogue having Arg and Ala or Gly respectively at positions corresponding to position 34 and position 8, respectively, of GLP-1(7-37) (SEQ ID NO: 1), and [Acy-(L1)ᵣ(L2)_{q}] is a substituent linked to the ε-amino group of the Lys residue at position 26 of the GLP-1 analogue, wherein
r is an integer from 1 to 10, and q is 0 or an integer from 1 to 10;
Acy is a fatty diacid containing 20-24 carbon atoms, wherein formally a hydroxyl group is removed from one of the carboxyl groups of the fatty diacid;
L1 is an acid residue selected from the following: γGlu, αGlu, βAsp, αAsp, γ-D-Glu, α-D-Glu, β-D-Asp, or α-D-Asp;
L2 is a neutral amino acid residue containing alkylene glycol;
Acy, L1, and L2 are connected by amide bonds; and
the order of L1 and L2 presented in formula (B) can be independently interchanged.

2. The pharmaceutical composition according to claim 1, wherein
G1 is [Gly8, Arg34]GLP-1-(7-37) peptide (SEQ ID NO: 2) or [Arg34]GLP-1-(7-37) peptide (SEQ ID NO: 3), preferably [Gly8, Arg34]GLP-1-(7-37) peptide; and/or
r is 1, 2, 3, 4, 5, or 6, preferably 1, 2, 3, or 4, preferably 1 or 2; preferably 1; and/or
q is 0, 1, 2, 3, 4, 5, 6, 7, or 8, preferably 0, 1, 2, 3, or 4, more preferably 0, 1, or 2; and/or
Acy is a fatty diacid containing 20-23 carbon atoms, preferably Acy is a fatty diacid containing 20, 21, or 22 carbon atoms.

3. The pharmaceutical composition according to any one of claims 1-2, wherein
L2 is: -HN-(CH₂)₂-O-(CH₂)₂-O-CH₂-CO-, -HN-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-CO-. - HN-(CH₂)₂-O-(CH2)2-O-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₃-O-(CH₂)₄-O-(CH2)3-NH-CO-, -HN-(CH₂)₃-O-(CH₂)₄-O-(CH₂)₃-NH-CO-CH₂-O-CH₂-CO-, -HN-(CH2)3-O-(CH₂)₄-O-(CH₂)₃-NH-CO-(CH₂)2-CO-, -HN-(CH₂)₂-O-(CH₂)2-O-CH₂-CO-CH₂-O-CH₂-CO-, -HN-(CH₂)₃-O-(CH₂)₂-O-(CH₂)₂-O-(CH₂)₃-NH-CO-(CH₂)₂-CO- , -HN-(CH₂)₃-O-(CH₂)₂-O-(CH₂)₂-O-(CH₂)₃-NH-CO-CH₂-O-CH₂-CO- , -HN-(CH₂)₂-O-(CH₂)₂-O-(CH₂)₂-NH-CO-(CH₂)₂-CO- , -HN-(CH₂)₂-O-(CH₂)₂-O-(CH₂)₂-NH-CO-CH₂O-CH₂-CO- , -HN-(CH₂)₃-O-(CH₂)₂-O-(CH₂)₂-O-(CH₂)₃-NH-CO-CH₂-O-CH₂-CO- , - HN-(CH₂)₃-O-(CH₂)₃-O-CH₂-CO-, or -HN-(CH₂)₄-O-(CH₂)₄-O-CH₂-CO-; preferably L2 is - HN-(CH₂)₂-O-(CH₂)₂-O-CH₂-CO-; and/or
L1 is selected from γGlu or βAsp, preferably L1 is γGlu; and/or
Acy is HOOC-(CH₂)₁₈-CO-, HOOC-(CH₂)₁₉-CO-, HOOC-(CH₂)₂₀-CO-, HOOC-(CH₂)₂₁-CO- or HOOC-(CH₂)₂₂-CO-, preferably, Acy is HOOC-(CH₂)₁₈-CO-.. HOOC-(CH₂)₂₀-CO- or HOOC-(CH₂)₂₂-CO-.

4. The pharmaceutical composition according to any one of claims 1-3, wherein Acy, L1, and L2 are sequentially connected by amide bonds in formula (B), and the C-terminus of L2 is connected to the ε-amino group of the Lys residue at position 26 of the GLP-1 analogue.

5. The pharmaceutical composition according to any one of claims 1-4, wherein the GLP-1 compound is selected from the following group consisting of
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl] [Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-21-carboxyheneicosanoylamino)-4(*S*)-carboxybutanoylamino]ethoxy)ethoxy]acetylamino)ethoxy]ethoxy)acetyl] [Gly8, Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Gly8, Arg34]GLP-l-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl] [Gly8, Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Gly8, Arg34]GLP-1-(7-37) peptide, *N*-ε²⁶-(23-carboxytricosanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(21-carboxyheneicosanoylamino)-4(*S*)-carboxybutanoyl-[Gly8, Arg34]GLP-1-(7-37) peptide,
*N*-ε²⁶-[2-(2-[2-(2-[2-(2-[4-(19-carboxynonadecanoylamino)-4(*S*)-carboxybutanoy1amino]ethoxy)ethoxy]acetylamino)ethoxy]ethoxy)acetyl][Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Arg34]GLP-1-(7-37)peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Arg34]GLP-1-(7-37) peptide,
*N*-ε²⁶-(23-carboxytricosanoylamino)-4(*S*)-carboxybutanoyl-[Arg34]GLP-1 - (7-37) peptide,
*N*-ε26-(19-carboxynonadecanoylamino)-4(*S*)-carboxybutanoyl-[Arg34]GLP-1-(7-37) peptide,
*N*-ε²⁶-(21-carboxyheneicosanoylamino)-4(*S*)-carboxybutanoyl-[Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl] [Gly8, Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Gly8, Arg34]GLP-1-(7-37)peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Gly8, Arg34]GLP-1-(7-37) peptide,
*N*-ε²⁶-(20-carboxyeicosanoylamino)-4(*S*)-carboxybutanoyl-[Gly8, Arg34]GLP-1-(7-37) peptide,
*N*-ε²⁶-(22-carboxydocosanoylamino)-4(*S*)-carboxybutanoyl-[Gly8, Arg34]GLP-l-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl] [Arg34]GLP-1 -(7-37) peptide,
*N*-ε²⁶-[2-(2-[2-(4-[20-carboxyeicosanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Arg34]GLP-1-(7-37) peptide,
*N*-ε²⁶-(20-carboxyeicosanoylamino)-4(*S*)-carboxybutanoyl-[Arg34]GLP-1-(7-37) peptide, and
*N*-ε²⁶-(22-carboxydocosanoylamino)-4(*S*)-carboxybutanoyl-[Arg34]GLP-1-(7-37) peptide;
preferably, the compound is selected from the following group consisting of
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl-[Arg34]GLP-1-(7-37) peptide,
*N*-ε²⁶-[2-(2-[2-(2-[2-(2-[4-(21-carboxyheneicosanoylainino)-4(*S*)-carboxybutanoylamino]ethoxy)ethoxy]acetylamino)ethoxylethoxy)acetyl][Glyg, Arg34]GLP-1-(7-37) peptide, and
*N*-ε²⁶-[2-(2-[2-(4-[21-carboxyheneicosanoylamino]-4(*S*)-carboxybutanoylamino)ethoxy]ethoxy)acetyl][Gly8, Arg34]GLP-1-(7-37) peptide.

6. The pharmaceutical composition of any one of claims 1-5, wherein the salt of N-(8-(2-hydroxybenzoyl)amino)caprylic acid (NAC) is sodium N-(8-(2-hydroxybenzoyl)amino)caprylate (SNAC).

7. The pharmaceutical composition of any one of claims 1-6, wherein the composition further comprises one or more pharmaceutically acceptable excipients.

8. The pharmaceutical composition of any one of claims 1-7, wherein the pharmaceutically acceptable excipient is selected from one or more from the group consisting of glidants, binders, fillers, disintegrants and lubricants.

9. The pharmaceutical composition of any one of claims 1-8, wherein the glidants is selected from talc and colloidal silica, preferably colloidal silica; and/or
the binder is selected from polyvinylpyrrolidone, copovidone and hydroxypropyl cellulose, preferably polyvinylpyrrolidone, more preferably polyvinylpyrrolidone K90; and/or
the filler is selected from the one or more from the group consisting of microcrystalline cellulose, cellulose powder, calcium hydrogen phosphate, corn starch, pregelatinized starch, anhydrous lactose, mannitol, erythritol, sucrose, sorbitol, calcium phosphate and dextrin, preferably microcrystalline cellulose or anhydrous lactose, more preferably microcrystalline cellulose; and/or
the lubricant is selected from the one or more from the group consisting of magnesium stearate, magnesium lauryl sulfate, stearic acid, sodium octadecyl fumarate and glyceryl tribehenate, preferably magnesium stearate.

10. The pharmaceutical composition of any one of claims 1-9, comprising:
at least about 50% (w/w), preferably at least about 55% (w/w), preferably at least about 60% (w/w), preferably about 50%-90% (w/w), preferably about 55%-85% (w/w), preferably about 60%-80% (w/w), preferably about 61% (w/w), about 62% (w/w), about 63% (w/w), about 64% (w/w), about 65% (w/w), about 66% (w/w), about 67% (w/w), about 68% (w/w), about 69% (w/w), about 70% (w/w), about 71% (w/w), about 72% (w/w), about 73% (w/w), about 74% (w/w), about 75% (w/w), about 76% (w/w), about 77% (w/w), about 78% (w/w), or about 79% (w/w) of the salt of NAC, preferably the salt of NAC is SNAC; and/or
not more than 25%, preferably not more than 20% (w/w), preferably not more than 17% (w/w), preferably not more than 15% (w/w), preferably about 0.5%-20% (w/w), preferably about 0.5%-18.5% (w/w), preferably about 0.5%-16.5% (w/w), preferably about 0.5%-15% (w/w), preferably about 0.50/0-13% (w/w), preferably about 0.50/0-12% (w/w), preferably about 0.5%-11% (w/w), preferably about 0.6%-1 1% (w/w), preferably about 0.6%-10% (w/w), preferably about 0.6%-9% (w/w), preferably about 0.6%-8% (w/w), preferably about 0.6%-7% (w/w), preferably about 0.6%-6% (w/w), preferably about 0.7%-5% (w/w), preferably about 1.2%-11% (w/w), preferably about 1.2%-10% (w/w), preferably about 1.2%-9% (w/w), preferably about 1.2%-8% (w/w), preferably about 1.20/0-7% (w/w), preferably about 1.2%-6% (w/w), preferably about 1.2%-5% (w/w), preferably about 1.2%-4.5% (w/w), preferably about 1.4%-4% (w/w), preferably about 1.4%-3.5% (w/w), preferably about 1.40/0-3% (w/w), preferably about 1.5% (w/w), about 1.7% (w/w), about 2% (w/w), about 2.5% (w/w), or about 2.8% (w/w) of the GLP-1 compound, preferably the GLP-1 compound is selected from the group consisting of *N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(21-carboxyhencicosanoylamino)-4(*S*)-carboxybutanoylamino]ethoxy)ethoxyjacetylamino)ethoxy]ethoxy)acetyl][Gly8, Arg34]GLP-1 - (7-37) peptide,
*N*-ε²⁶-[2-(2-[2-(4-[21-carboxyhcneicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Gly8, Arg34]GLP-1-(7-37) peptide,
*N*-ε²⁶-(23-carboxytricosanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(21-carboxyheneicosanoylamino)-4(*S*)-carboxybutanoyl-[Gly8, Arg34]GLY-1-(7-37) peptide,
*N*-ε²⁶-[2-(2-[2-(2-[2-(2-[4-(19-carboxynonadecanoylamino)-4(*S*)-carboxybutanoylamino]ethoxy)cthoxy]acetylamino)ethoxy]ethoxy)acetyl][Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Arg34]GLP-1-(7-37) peptide,
*N*-ε²⁶-(23-carboxytricosanoylamino)-4(*S*)-carboxybutanoyl-[Arg34]GLP-1-(7-37) peptide,
*N*-ε²⁶-(19-carboxynonadecanoylamino)-4(*S*)-carboxybutanoyl-[Arg34]GLP-1-(7-37) peptide,
*N*-ε²⁶-(21-carboxyheneicosanoylamino)-4(*S*)-carboxybutanoyl-[Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Gly8, Arg34]GLP-1 - (7-37) peptide,
*N*-e²⁶-[2-(2-[2-(4-[22-carboxydocosanoylamino]-4(*S*)-carboxybutanoylamino)ethoxy]ethoxy)acetyl][Gly8, Arg34]GLP-1-(7-37) peptide,
*N*-ε²⁶-(20-carboxyeicosanoylamino)-4(*S*)-carboxybutanoyl-[Gly8, Arg34]GLP-1-(7-37) peptide,
*N*-e²⁶-(22-carboxydocosanoylamino)-4(*S*)-carboxybutanoyl-[Gly8, Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Arg34]GLP-1 -(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Arg34]GLP-1-(7-37) peptide,
*N*-ε²⁶-(20-carboxyeicosanoylamino)-4(*S*)-carboxybutanoyl-[Arg34]GLP-1-(7-37) peptide, and
*N*-ε²⁶-(22-carboxydocosanoylamino)-4(*S*)-carboxybutanoyl-[Arg34]GLP-1-(7-37) peptide.

11. The pharmaceutical composition of any one of claims 1-10, further comprising:
about 0.1%-20% (w/w), preferably about 0.2%-10% (w/w), preferably about 0.3%-5% (w/w), preferably about 0.5%-3% (w/w), preferably about 0.7%-2% (w/w), preferably about 1%-2% (w/w), preferably about 1.6% (w/w), about 2.0% (w/w), preferably about 1.6% (w/w), about 1.7% (w/w), about 1.8% (w/w), about 1.9% (w/w), or about 2.0% (w/w) of the binder, preferably the binder is selected from polyvinylpyrrolidone, copovidone, and hydroxypropyl cellulose, preferably polyvinylpyrrolidone, more preferably polyvinylpyrrolidone K90; and/or
about 5%-40% (w/w), preferably about 10%-35% (w/w), preferably about 10%-30% (w/w), preferably about 10%-25% (w/w), preferably about 13%-17% (w/w), preferably about 13% (w/w), about 14% (w/w), about 15% (w/w), about 16% (w/w), about 17% (w/w), or about 20% (w/w) of the filler, wherein the filler is selected from microcrystalline cellulose, cellulose powder, calcium hydrogen phosphate, corn starch, pregelatinized starch, anhydrous lactose, mannitol, erythritol, sucrose, sorbitol, calcium phosphate, and dextrin, preferably microcrystalline cellulose or anhydrous lactose, more preferably microcrystalline cellulose; and/or
about 0.1%-10% (w/w), preferably about 0.5%-10% (w/w), preferably about 0.5%-5% (w/w), preferably about 0.5%-3.5% (w/w), preferably about 2.8% (w/w), about 2.9% (w/w), about 3% (w/w), about 3.1% (w/w), about 3.2% (w/w), about 3.3% (w/w), about 3.4% (w/w), or about 3.5% (w/w) of the lubricant, wherein the lubricant is selected from magnesium stearate, magnesium lauryl sulfate, stearic acid, sodium stearyl fumarate, and glyceryl tribehenate, preferably magnesium stearate; and/or
about 0.1%-20% (w/w), preferably about 0.2%-10% (w/w), preferably about 0.3%-5% (w/w), preferably about 0.5%-3% (w/w), preferably about 0.7%-2% (w/w), preferably about 1%-2% (w/w), preferably about 1.6%-2% (w/w), preferably about 1.6% (w/w), about 1.7% (w/w), about 1.8% (w/w), about 1.9% (w/w), or about 2% (w/w) of the glidant, wherein the glidants is selected from talc, colloidal silica, preferably colloidal silica.

12. The pharmaceutical composition of any one of claims 1-11, comprising:
about 1-100 mg, preferably about 2-90 mg, preferably about 3-80 mg, preferably about 4-60 mg, preferably about 5-55 mg, preferably about 5-50 mg, preferably about 5-45 mg, preferably about 5-40 mg, preferably about 5-35 mg, preferably about 5-30 mg, preferably about 5-25 mg, preferably about 5-20 mg, preferably about 5-15 mg, preferably about 5-10 mg, preferably about 7-50 mg, preferably about 7-30 mg, preferably about 7-25 mg, preferably about 7-20 mg, preferably about 2 mg, about 3 mg, about 4 mg, about 5 mg, about 6 mg, about 7 mg, about 8 mg, about 9 mg, about 10 mg, about 14 mg, about 25 mg, or about 50 mg of the GLP-1 compound, preferably the GLP-1 compound is selected from the goup consisting of *N*-ε²⁶-[2-(2-[2-(2-[2-(2-[4-(19-carboxynonadecanoylamino)-4(5)-carboxybutanoylamino]ethoxy)ethoxy]acetylamino)ethoxy]ethoxy)acetyl] [Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly8, Arg34]GLP-l-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Gly8, Arg34]GLP-l-(7-37) peptide,
*N*-ε²⁶-(23-carboxytricosanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(21-carboxyheneicosanoylamino)-4(*S*)-carboxybutanoyl-[Gly8, Arg34]GLP-1-(7-37) peptide,
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acety1][Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Arg34]GLP-1 -(7-37) peptide, *N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acety1][Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Arg34]GLP-1-(7-37) peptide,
*N*-ε²⁶-(23-carboxytricosanoylamino)-4(*S*)-carboxybutanoyl-[Arg34]GLP-1-(7-37) peptide,
*N*-ε²⁶-(19-carboxynonadecanoylamino)-4(*S*)-carboxybutanoyl-[Arg34]GLP-1-(7-37) peptide,
*N*-ε²⁶-(21-carboxyheneicosanoylamino)-4(*S*)-carboxybutanoyl-[Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Gly8, Arg34]GLP-1-(7-37) peptide,
*N*-_{ε}²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Gly8, Arg34]GLP-1 - (7-37) peptide,
*N*-ε²⁶-[2-(2-[2-(4-[22-carboxydocosanoylamino]-4(*S*)-carboxybutanoylamino)ethoxy]ethoxy)acetyl] [Gly 8, Arg34]GLP-1-(7-37) peptide, *N*-ε²⁶-(20-carboxyeicosanoylamino)-4(*S*)-carboxybutanoyl-[Gly8, Arg34]GLP-1-(7-37) peptide, *N*-ε²⁶-(22-carboxydocosanoylamino)-4(*S*)-carboxybutanoyl-[Gly8, Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Arg34]GLP-1-(7-37) peptide,
*N*-e²⁶-(20-carboxyeicosanoylamino)-4(*S*)-carboxybutanoyl-[Arg34]GLP-1-(7-37) peptide, and
*N*-ε²⁶-(22-carboxydocosanoylamino)-4(*S*)-carboxybutanoyl-[Arg34]GLP-1 - (7-37) peptide; and/or
at least about 50 mg, preferably at least about 100 mg, preferably at least about 150 mg, preferably about 50-600 mg, preferably about 100-500 mg, preferably about 150-450 mg, preferably about 175-425 mg, preferably about 200-400 mg, preferably about 250-400 mg, more preferably about 300 mg, 350 mg, 400 mg of the salt of NAC, wherein the salt of NAC is SNAC; and/or
about 0.5-50 mg, preferably about 1-40 mg, preferably about 1-30 mg, preferably about 1-25 mg, preferably about 1-20 mg, preferably about 1-15 mg, preferably about 2-14 mg, preferably about 3-13 mg, preferably about 4-12 mg, preferably about 5-11 mg, preferably about 6-10 mg, preferably about 7-9 mg, preferably about 8 mg of a binder, wherein the binder is selected from polyvinylpyrrolidone, copovidone, and hydroxypropyl cellulose, preferably polyvinylpyrrolidone, more preferably polyvinylpyrrolidone K90; and/or
about 10-150 mg, preferably about 20-140 mg, preferably about 30-130 mg, preferably about 40-120 mg, preferably about 50-110 mg, preferably about 50-100 mg, preferably about 60-90 mg, preferably about 60-80 mg, preferably about 60-70 mg, preferably about 68 mg of a filler, wherein the filler is selected from microcrystalline cellulose, cellulose powder, calcium hydrogen phosphate, corn starch, pregelatinized starch, anhydrous lactose, mannitol, erythritol, sucrose, sorbitol, calcium phosphate, and dextrin, preferably microcrystalline cellulose or anhydrous lactose, more preferably microcrystalline cellulose; and/or
about 1-50 mg, preferably about 1-40 mg, preferably about 1-30 mg, preferably about 2-20 mg, preferably about 3-20 mg, preferably about 5-20 mg, preferably about 5-15 mg, preferably about 10-15 mg, preferably about 14 mg of a lubricant, wherein the lubricant is selected from magnesium stearate, magnesium lauryl sulfate, stearic acid, sodium stearyl fumarate, and glyceryl tribehenate, preferably magnesium stearate; and/or
about 1-50 mg, preferably about 1-40 mg, preferably about 1-30 mg, preferably about 2-20 mg, preferably about 3-20 mg, preferably about 5-15 mg, preferably about 5-10 mg, preferably about 8 mg of a glidant, wherein the glidant is selected from talc and colloidal silica, preferably colloidal silica.

13. A pharmaceutical composition according to any one of claims 1-12, wherein the pharmaceutical composition is in the form of a tablet, granule, or capsule formulation, preferably a tablet formulation.

14. A pharmaceutical composition according to any one of claims 1-13, wherein the hardness is less than about 130N, preferably about 80-130N, preferably about 90-130N, preferably about 100-125N, more preferably about 110-120N.

15. A pharmaceutical composition according to any one of claims 1-14, wherein the GLP-1 compound has a particle size D90 preferably not exceeding about 200 µm, preferably not exceeding about 130 µm, preferably being about 15-130 µm, more preferably being about 15-50 µm.

16. A pharmaceutical composition comprising:
about 5, 10, 15, 20, 25, 30, 35, 40, 45, 50, 55, 60, 70, 80, 90, or 100 mg, preferably about 7-10 mg of *N*-ε²⁶-[2-(2-[2-(2-[2-(2-[4-(21-carboxyheneicosanoylamino)-4(*S*)-carboxybutanoylamino]ethoxy)ethoxy]acetylamino)ethoxyJethoxy)acetyl][Gly8, Arg34]GLP-1 - (7-37) peptide;
about 300, 350, or 400 mg of SNAC;
about 8 mg of polyvinylpyrrolidone K90;
about 68 mg of microcrystalline cellulose;
about 14 mg of magnesium stearate; and
about 8 mg of colloidal silica.

17. A pharmaceutical composition according to any one of claims 1-16, wherein the total weight of the pharmaceutical composition is about 150-1000 mg, preferably about 175-1000mg, preferably about 200-800mg, preferably about 400mg-500mg, preferably about 400mg, about 403mg, about 405 mg, about 408 mg, about 413mg, about 418mg, about 423mg, about 428mg, about 433mg, about 438mg, about 443mg, about 448mg, about 455mg, about 500mg, about 600mg, or about 800 mg.

18. A method of preparing a pharmaceutical composition according to any one of claims 1-17, comprising a step of one-time granulation after mixing the GLP-1 compound with the salt of NAC.

19. The method according to claim 18, wherein the salt of NAC is mixed with a lubricant and/or a glidant before mixing with the GLP-1 compound.

20. The method of preparing a pharmaceutical composition according toany one of claims 1-17, comprising:
(1) mixing the salt of NAC, the glidant, and a portion of the lubricant to obtain a premix powder A;
(2) mixing the GLP-1 compound, the binder, and the filler with the premix powder A to obtain a premix powder B;
(3) granulating the premix powder B, preferably by dry granulation;
(4) mixing the obtained granules with the remaining lubricant to obtain a blend of granules;
(5) compressing the blend of granules into tablets.

21. A method according to claims 18-20, wherein the GLP-1 compound is selected from *N*-ε²⁶-[2-(2-[2-(2-[2-(2-[4-(19-carboxynonadecanoylamino)-4(S)-carboxybutanoylamino]ethoxy)ethoxy]acetylamino)ethoxy]ethoxy)acetyl] [Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Gly8, Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Gly8, Arg34]GLP-1-(7-37) peptide,
*N*-ε²⁶-(23-carboxytricosanoylamino)-4(5)-carboxybutanoyl-[Gly8, Arg34]GLP-l-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(21-carboxyheneicosanoylamino)-4(*S*)-carboxybutanoyl-[Gly8, Arg34]GLP-1-(7-37) peptide,
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetylj[Arg34]GLP-1 -(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxyjethoxy)acetyl][Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl][Arg34]GLP-1-(7-37) peptide,
*N*-ε²⁶-(23-carboxytricosanoylamino)-4(*S*)-carboxybutanoyl-[Arg34]GLP-1 - (7-37) peptide,
*N*-ε²⁶-(19-carboxynonadecanoylamino)-4(*S*)-carboxybutanoyl-[Arg34]GLP-1-(7-37) peptide,
*N*-ε²⁶-(21-carboxyheneicosanoylamino)-4(*S*)-carboxybutanoyl-[Arg34]GLP-1-(7-37) peptide,
*N*-_{ε}²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Gly8, Arg34]GLP-1-(7-37) peptide,
*N*^{-ε26}-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Gly8, Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Gly8, Arg34]GLP-1-(7-37) peptide,
*N*-ε²⁶-(20-carboxyeicosanoylamino)-4(8)-carboxybutanoyl-[Gly8,Arg34]GLP-1-(7-37)peptide,
*N*-ε²⁶-(22-carboxydocosanoylamino)-4(*S*)-carboxybutanoyl-[Gly8, Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylainino)ethoxy]ethoxy)acetyl][Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl][Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl][Arg34]GLP-1-(7-37) peptide,
*N*-ε²⁶-(20-carboxyeicosanoylamino)-4(*S*)-carboxybutanoyl-[Arg34]GLP-1-(7-37) peptide, and
*N*-ε²⁶-(22-carboxydocosanoylamino)-4(*S*)-carboxybutanoyl-[Arg34]GLP-1 - (7-37) peptide; and/or
the salt of NAC is SNAC; and/or
the binder is selected from polyvinylpyrrolidone, copovidone, and hydroxypropyl cellulose, preferably polyvinylpyrrolidone, more preferably polyvinylpyrrolidone K90; and/or
the filler is selected from microcrystalline cellulose, cellulose powder, calcium hydrogen phosphate, corn starch, pregelatinized starch, anhydrous lactose, mannitol, erythritol, sucrose, sorbitol, calcium phosphate, and dextrin, preferably microcrystalline cellulose or anhydrous lactose, more preferably microcrystalline cellulose; and/or
the lubricant is selected from magnesium stearate, magnesium lauryl sulfate, stearic acid, sodium stearyl fumarate, and glyceryl tribehenate, preferably magnesium stearate; and/or
the glidant is selected from talc and colloidal silica, preferably colloidal silica.

22. A pharmaceutical composition according to any one of claims 1-17 for use as a medicament.

23. A pharmaceutical composition according to any one of claims 1-17, for use in the treatment or prevention of hyperglycemia, diabetes, and/or obesity.

24. Use of the pharmaceutical composition according to any one of claims 1-17 in the preparation of a medicament for the treatment or prevention of hyperglycemia, diabetes, and/or obesity.

25. A method for the treatment or prevention of hyperglycemia, diabetes, and/or obesity, comprising administering an effective amount of the pharmaceutical composition according to any one of claims 1-17.
